(19) Europäisches Patentamt
European Patent Office
Office européen des brevets

(11) Veröffentlichungsnummer: **0 300 218**
**A1**

# (12) EUROPÄISCHE PATENTANMELDUNG

(21) Anmeldenummer: 88109993.1

(22) Anmeldetag: 23.06.88

(51) Int. Cl.4: C07C 50/32 , C07C 46/00 , C07C 69/96 , C07C 69/145 , C07C 143/68 , C07F 7/08 , A01N 35/06 , A01N 37/02 , A01N 41/04

(30) Priorität: 03.07.87 DE 3722018
22.01.88 DE 3801743

(43) Veröffentlichungstag der Anmeldung:
25.01.89 Patentblatt 89/04

(84) Benannte Vertragsstaaten:
BE CH DE ES FR GB GR IT LI NL

(71) Anmelder: BAYER AG
Konzernverwaltung RP Patentabteilung
D-5090 Leverkusen 1 Bayerwerk(DE)

(72) Erfinder: Lindner, Werner, Dr.
Maerchenstrasse 39
D-5000 Köln 80(DE)
Erfinder: Becker, Benedikt, Dr.
Metzkausener Strasse 14
D-4020 Mettmann(DE)
Erfinder: Steffens, Robert, Dr.
Roggendorfstrasse 63
D-5000 Köln 80(DE)
Erfinder: Wachendorff-Neumann, Ulrike, Dr.
Hermann-Loens-Strasse 16
D-5090 Leverkusen 3(DE)
Erfinder: Brandes, Wilhelm, Dr.
Eichendorffstrasse 3
D-5653 Leichlingen(DE)
Erfinder: Stendel, Wilhelm, Dr.
In den Birken 55
D-5600 Wuppertal 1(DE)
Erfinder: Dutzmann, Stefan, Dr.
Leinenweberweg 33
D-4000 Düsseldorf 13(DE)

## (54) Schädlingsbekämpfungsmittel auf Basis von substituierten 1,4-Naphthochinonen und neue substituierte 1,4-Naphthochinone.

(57) Die Erfindung betrifft die Verwendung von teilweise bekannten substituierten 1,4-Naphthochinonen als Schädlingsbekämpfungsmittel, insbesondere als Akarizide und als Fungizide, sowie neue substituierte 1,4-Naphthochinone und Verfahren zu ihrer Herstellung.

Es wurde gefunden, daß die teilweise bekannten substituierten 1,4-Naphthochinone der allgemeinen Formel (I)

EP 0 300 218 A1

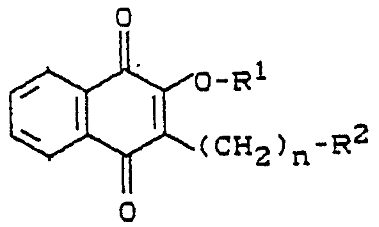

(I)

in welcher
n für die Zahlen Null oder 1 bis 12 steht,
$R^1$ für Wasserstoff oder für einen gegebenenfalls substituierten Rest R aus der Reihe Alkyl, Aralkyl, Alkylcarbonyl, Alkoxycarbonyl, Alkylsulfonyl und Arysulfonyl steht und
$R^2$ für Halogenalkyl, gegebenenfalls substituiertes Aryl oder substituiertes Cycloalkyl steht,
eine starke Wirkung als Schädlingsekämpfungsmittel, insbesondere als Akarizide und Fungizide, aufweisen.

# Schädlingsbekämpfungsmittel auf Basis von substituierten 1,4-Naphthochinonen und neu substituierte 1,4-Naphthochinone

Die Erfindung betrifft die Verwendung von teilweise bekannten substituierten 1,4-Naphthochinonen als Schädlingsbekämpfungsmittel, insbesondere als Akarizide und als Fungizide, sowie neue substituierte 1,4-Naphthochinone und Verfahren zu ihrer Herstellung.

Es ist bekannt, daß bestimmte substituierte 1,4-Naphthochinone, wie z. B. 2-Nonyl-, 2-Decyl- und 2-(3-Cyclohexyl-propyl)-3-hydroxy-1,4-naphthochinon, insektizide und akarizide Eigenschaften aufweisen (vgl. US-PS 2 572 946).

Auch eine fungizide Wirkung solcher Verbindungen ist bekannt geworden (vgl. Pestic. Sci. 4 (1973), 193 -200). Die Wirkung dieser Verbindungen ist jedoch, insbesondere bei niedrigen Aufwandmengen bzw. Wirkstoffkonzentrationen, nicht immer zufriedenstellend.

Es ist weiter bekannt, daß bestimmte substituierte 1,4-Naphthochinone, welche spezielle Cycloalkylgruppen enthalten, gegen Protozoen bei Nutztieren eingesetzt werden können (vgl. EP-A 2 228, EP-A 77 550, EP-A 77 551, EP-A 123 238 und EP-A 123 239). Über eine Verwendung solcher Verbindungen im Pflanzenschutz, z. B. als Akarizide bzw. als Fungizide, ist jedoch bisher nichts bekannt.

Es wurde nun gefunden, daß die teilweise bekannten substituierten 1,4-Naphthochinone der allgemeinen Formel (I)

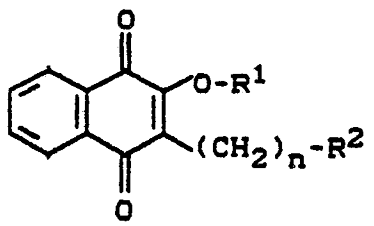

(I)

in welcher

n für die Zahlen Null oder 1 bis 12 steht,

$R^1$ für Wasserstoff oder für einen gegebenenfalls substituierten Rest R aus der Reihe Alkyl, Aralkyl, Alkylcarbonyl, (Hetero)Arylcarbonyl, Alkoxycarbonyl, Alkylsulfonyl und Arylsulfonyl steht und

$R^2$ für Halogenalkyl, gegebenenfalls substituiertes (Hetero)Aryl oder substituiertes Cycloalkyl steht,

starke Wirkung als Schädlingsbekämpfungsmittel, insbesondere als Akarizide und als Fungizide, aufweisen.

Überraschenderweise zeigen die substituierten 1,4-Naphthochinone der allgemeinen Formel (I) erheblich stärkere akarizide und fungizide Wirkung als die oben genannten bekannten Verbindungen ähnlicher Struktur.

Die Erfindung betrifft vorzugsweise die Verwendung von Schädlingsbekämpfungsmitteln und Schädlingsbekämpfungsmittel, insbesondere Akarizide und Fungizide, auf Basis von Verbindungen der allgemeinen Formel (I), in welcher

n für die Zahlen Null oder 1 bis 8 steht,

$R^1$ für Wasserstoff oder für einen gegebenenfalls wie nachstehend angegeben substituierten Rest R aus der Reihe $C_1$-$C_{15}$-Alkyl (welches gegebenenfalls durch Fluor, Chlor oder $C_3$-$C_6$-Cycloalkyl substituiert ist), Benzyl (welches gegebenenfalls durch Fluor, Chlor oder Brom substituiert ist), $C_1$-$C_{15}$-Alkyl-carbonyl (welches gegebenenfalls durch Fluor, Chlor, Brom, Phenyl, $C_1$-$C_2$-Alkoxy oder $C_3$-$C_6$-Cycloalkyl substituiert ist), Benzoyl (welches gegebenenfalls durch Fluor, Chlor, Brom, Iod, Cyano, Nitro, $C_1$-$C_4$-Alkyl, $C_1$-$C_4$-Alkoxy, $C_1$-$C_4$-Halogenalkyl und/oder $C_1$-$C_4$-Halogenalkoxy substituiert ist), Furylcarbonyl oder Thienylcarbonyl (welche gegebenenfalls durch $C_1$-$C_4$-Alkyl substituiert sind), $C_1$-$C_4$-Alkoxy-carbonyl, $C_1$-$C_8$-Alkylsulfonyl (welches gegebenenfalls durch Fluor oder Chlor substituiert ist) und Phenylsulfonyl oder Naphthylsulfonyl (welche gegebenenfalls durch Fluor, Chlor, Brom, Cyano, Nitro, $C_1$-$C_4$-Alkyl, Trifluormethyl, $C_1$-$C_4$-Alkoxy, $C_1$-$C_4$-Alkylsulfonyl, Dimethylaminosulfonyl und/oder $C_1$-$C_4$-Alkoxy-carbonyl substituiert sind) steht und

$R^2$ für durch Fluor und/oder Chlor substituiertes $C_1$-$C_8$-Alkyl, gegebenenfalls durch Fluor, Chlor, Brom, Cyano, Nitro, $C_1$-$C_6$-Alkyl, Trifluormethyl, $C_1$-$C_4$-Alkoxy, $C_1$-$C_2$-Fluoralkoxy, $C_1$-$C_4$-Alkylthio, $C_1$-$C_2$-Fluoralkylthio, $C_1$-$C_4$-Alkylsulfinyl, $C_1$-$C_2$-Fluoralkylsulfinyl, $C_1$-$C_4$-Alkylsulfonyl, $C_1$-$C_2$-Fluoralkylsulfonyl, Phenyl und/oder Tri-($C_1$-$C_2$-alkyl)-silyl substituiertes Phenyl, für Naphthyl, für gegebenenfalls durch $C_1$-$C_4$-Alkyl

substituiertes Furyl oder Thienyl oder für durch $C_1$-$C_6$-Alkyl, Trifluormethyl, Cyclohexyl und/oder Tri($C_1$-$C_2$-alkyl)-silyl substituiertes Cyclohexyl steht.

Besonders bevorzugt werden erfindungsgemäß die Verbindungen der Formel (I) verwendet, in welcher n für die Zahlen Null oder 1 bis 4 steht,
$R^1$ für Wasserstoff, Methyl oder Acetyl steht und
$R^2$ für Perfluor-$C_1$-$C_6$-Alkyl, für durch Fluor, Chlor, $C_1$-$C_4$-Alkyl, Trifluormethyl, $C_1$-$C_3$-Alkoxy, Trifluormethoxy, $C_1$-$C_3$Alkylthio, Trifluormethylsulfenyl, $C_1$-$C_3$-Alkylsulfinyl, Trifluormethylsulfinyl, $C_1$-$C_3$-Alkylsulfonyl, Trifluormethylsulfonyl oder Trimethylsilyl substituiertes Phenyl oder für durch $C_1$-$C_4$-Alkyl, Trifluormethyl oder Trimethylsilyl substituiertes Cyclohexyl steht.

Als Beispiele für die erfindungsgemäß zu verwendenden Verbindungen der Formel (I) seien genannt: 2-(4-tert-Butyl-cyclohexyl)j-3-hydroxy-1,4-naphthochinon, 2-(4-Trimethylsilyl-cyclohexyl)-3-hydroxy-1,4-naphthochinon, 2-(3-Trifluormethyl-1-cyclohexyl-methyl)-3-hydroxy-1,4-naphthochinon, 2-(4-Trifluormethyl-1-cyclohexylmethyl)-3-hydroxy-1,4-naphthochinon, 2-(2-(4-Trifluormethyl-1-cyclohexyl)-ethyl)-3-hydroxy-1,4-naphthochinon, 2-(3-Trifluormethyl-1-cyclohexyl-methyl)-3-acetoxy-1,4-naphthochinon, 2-(3-Trifluormethyl-phenylmethyl)-3-hydroxy-1,4-naphthochinon und 2-(2-(4-Trifluormethylthio-phenyl)-ethyl)-3-hydroxy-1,4-naphthochinon.

Diese Verbindungen werden wegen ihrer hervorragenden akariziden und fungiziden Wirkung ganz besonders bevorzugt.

Weiter sind Beispiele für die erfindungsgemäß zu verwendenen Verbindungen der Formel (I) in der nachstehenden Tabelle 1 aufgeführt.

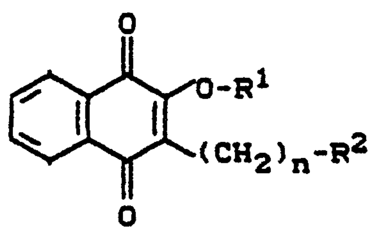

(I)

**Tabelle 1: Beispiele für Verbindungen der Formel (I)**

| Bsp.-Nr. | $R^1$ | $R^2$ | n | Schmelzpunkt (°C) |
|---|---|---|---|---|
| 1 | H | 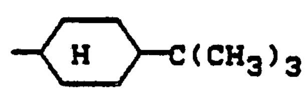 | 0 | 95 |

Tabelle 1 - Fortsetzung

| Bsp.-Nr. | $R^1$ | $R^2$ | n | Schmelzpunkt (°C) |
|---|---|---|---|---|
| 2 | H | 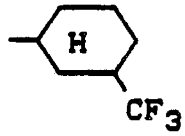 | 1 | 142 |
| 3 | H | 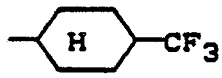 | 1 | 125 |
| 4 | H | 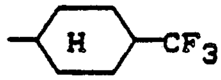 | 2 | 115 |
| 5 | H | 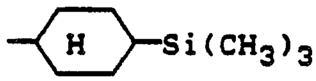 | 0 | 65 |
| 6 | H | 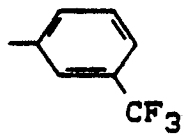 | 1 | 158 |
| 7 | H | 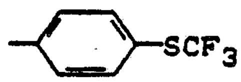 | 2 | 118 |
| 8 | H | $CF_3$ | 2 | 140 |
| 9 | $-CO-CH_3$ | 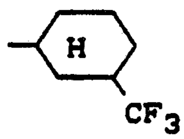 | 1 | 110 |
| 10 | $-CO-OCH_3$ | 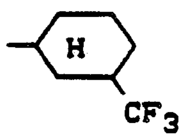 | 1 | 114 |
| 11 | 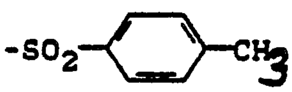 | 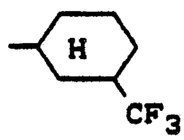 | 1 | 116 |

Tabelle 1 - Fortsetzung

| Bsp.-Nr. | $R^1$ | $R^2$ | n | Schmelzpunkt (° C) |
|---|---|---|---|---|
| 12 | $-CH_2-$ 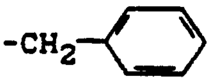 | 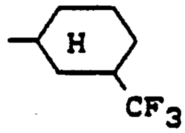 | 1 | 100 |
| 13 | $-CO-CH_2CH_2CCl_2CF_3$ | 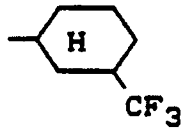 | 1 | (amorph) |
| 14 | $-CO-CH_3$ | 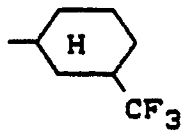 | 0 | 110 |
| 15 | $-CH_3$ | 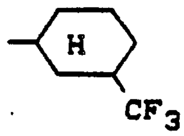 | 1 | 90 |
| 16 | $-CO-CH_3$ | 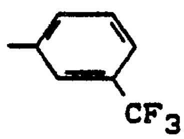 | 1 | 108 |
| 17 | $-CO-CH_3$ | 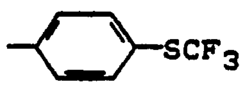 | 2 | 105 |
| 18 | $-CO-CH_3$ | $CF_3$ | 2 | 105 |
| 19 | $-SO_2-CH_3$ | 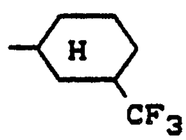 | 1 | |
| 20 | $-CO-CH_2Cl$ | 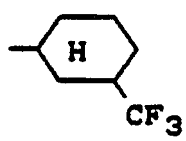 | 1 | |
| 21 | $-CO-CHCl_2$ | 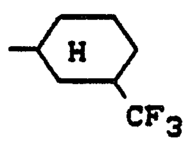 | 1 | |

<u>Tabelle 1</u> - Fortsetzung

| Bsp.-Nr. | $R^1$ | $R^2$ | n | Schmelzpunkt (°C) |
|---|---|---|---|---|
| 22 | $-SO_2CF_3$ | 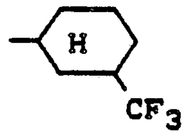 | 1 | |
| 23 | $-\underset{\underset{O}{\Vert}}{C}-(CH_2)_7CH_3$ | 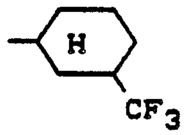 | 1 | |
| 24 | H | 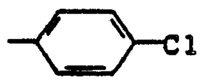 | 2 | |
| 25 | H | 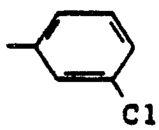 | 2 | |
| 26 | H | 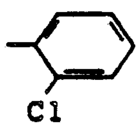 | 2 | 171 |
| 27 | H | 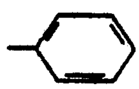 | 2 | 177 |
| 28 | H | 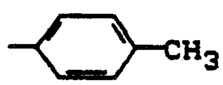 | 1 | 168 |
| 29 | H | 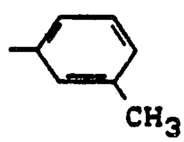 | 2 | |
| 30 | H | 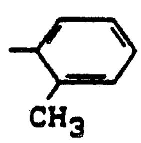 | 2 | |

Tabelle 1 - Fortsetzung

| Bsp.-Nr. | $R^1$ | $R^2$ | n | Schmelzpunkt (°C) |
|---|---|---|---|---|
| 31 | H | 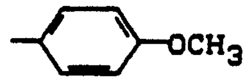 | 2 | |
| 32 | H | 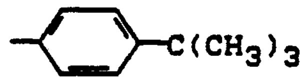 | 2 | 105 |
| 33 | $-\underset{\underset{O}{\Vert}}{C}-CH_3$ | 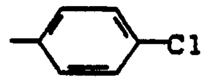 | 2 | |
| 34 | $-\underset{\underset{O}{\Vert}}{C}-CH_3$ | 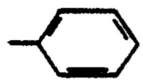 | 2 | 93 |
| 35 | $-\underset{\underset{O}{\Vert}}{C}-CH_3$ | 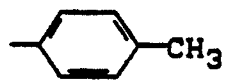 | 2 | |
| 36 | $-CH_3$ | 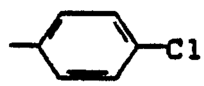 | 2 | |
| 37 | $-CH_3$ | 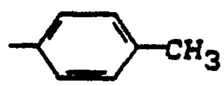 | 2 | |
| 38 | $-CH_2$ 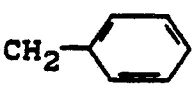 | 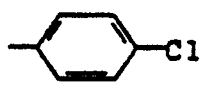 | 2 | |
| 39 | H | $-(CH_2)_7-CF_3$ | 1 | |
| 40 | $-\underset{\underset{O}{\Vert}}{C}-CH_3$ | $-(CH_2)_7-CF_3$ | 1 | |

Tabelle 1 - Fortsetzung

| Bsp.-Nr. | $R^1$ | $R^2$ | n | Schmelzpunkt (° C) |
|---|---|---|---|---|
| 41 | $-CH_3$ | $-(CH_2)_7-CF_3$ | 1 | |
| 42 | $-CH_2$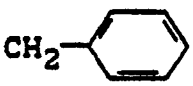 | $-(CH_2)_7-CF_3$ | 1 | |
| 43 | $-SO_2$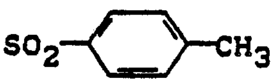 | $-(CH_2)_7-CF_3$ | 1 | |
| 44 | H | 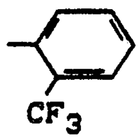 | 2 | 105 |
| 45 | H | 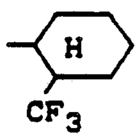 | 2 | 88 |
| 46 | $-CO-CH_3$ | 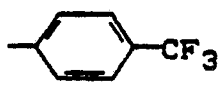 | 1 | 160 |
| 47 | $-CO-CH_3$ | 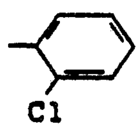 | 2 | 90 |
| 48 | $-CO-CH_3$ | 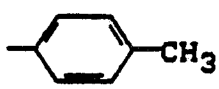 | 1 | 92 |
| 49 | H | 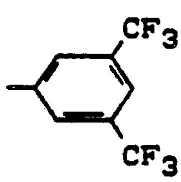 | 2 | 142 |

<u>Tabelle 1</u> - Fortsetzung

| Bsp.-Nr. | $R^1$ | $R^2$ | n | Schmelzpunkt (°C) |
|---|---|---|---|---|
| 50 | H | 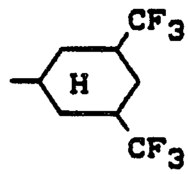 | 2 | 120 |
| 51 | $-CO-CH_3$ | 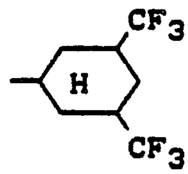 | 2 | 97 |
| 52 | $-CO-CH_3$ | 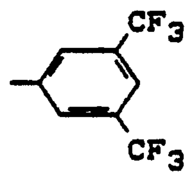 | 2 | 107 |
| 53 | $-CO-CH_3$ | 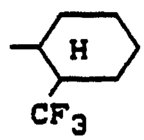 | 2 | |
| 54 | $-CO-CH_3$ | 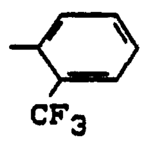 | 2 | 103 |
| 55 | H | 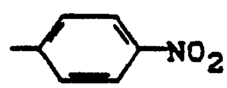 | 1 | 251 |
| 56 | $-CO-CH_3$ | 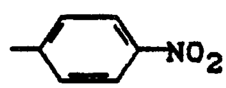 | 1 | 128 |
| 57 | H | 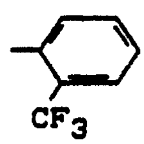 | 0 | 175 |

Tabelle 1 - Fortsetzung

| Bsp.-Nr. | $R^1$ | $R^2$ | n | Schmelzpunkt (° C) |
|---|---|---|---|---|
| 58 | H | 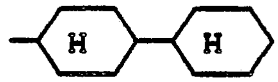 | 0 | 170 |
| 59 | H | 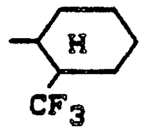 | 0 | 95 |
| 60 | H | 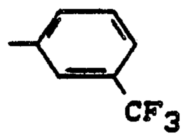 | 2 | 178 |
| 61 | H | 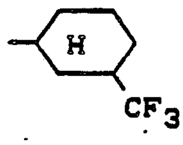 | 2 | 130 |
| 62 | $-CO-CH_3$ | 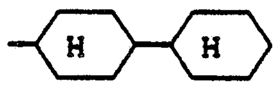 | 0 | 125 |
| 63 | $-CO-CH_3$ | 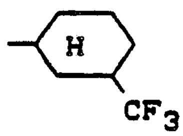 | 2 | 55 |
| 64 | $-CO-CH_3$ | 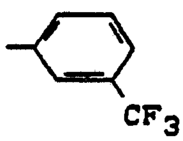 | 2 | 77 |
| 65 | $-CO-CH_3$ | 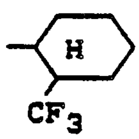 | 0 | (amorph) |
| 66 | $-CO-CH_3$ | 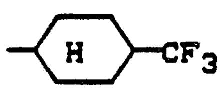 | 1 | 90 |

Tabelle 1 - Fortsetzung

| Bsp.-Nr. | $R^1$ | $R^2$ | n | Schmelzpunkt (°C) |
|---|---|---|---|---|
| 67 | H | $CF_3$ | 4 | 102 |
| 68 | H | 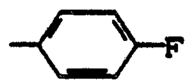 | 1 | 158 |
| 69 | H | 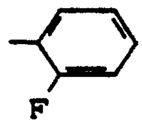 | 1 | 125 |
| 70 | $-CO-CH_3$ | $C_6F_{13}$ | 2 | 60 |
| 71 | H | 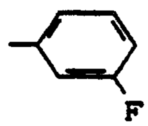 | 1 | 178 |
| 72 | H | 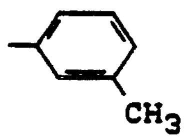 | 1 | 125 |
| 73 | H | 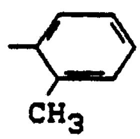 | 1 | 162 |
| 74 | $-CO-CH_3$ | $CF_3$ | 4 | 53 |
| 75 | $-CO-CH_3$ | 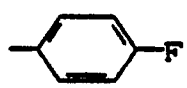 | 1 | 150 |
| 76 | $-CO-CH_3$ | 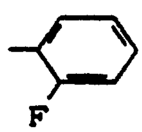 | 1 | 120 |

Tabelle 1 - Fortsetzung

| Bsp.-Nr. | $R^1$ | $R^2$ | n | Schmelzpunkt (°C) |
|---|---|---|---|---|
| 77 | $-CO-CH_3$ | 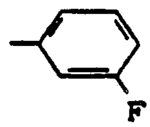 | 1 | 80 |
| 78 | $-CO-CH_3$ | 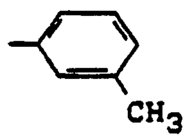 | 1 | 55 |
| 79 | $-CO-CH_3$ | 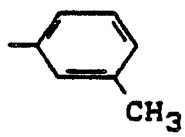 | 1 | 112 |
| 80 | H | 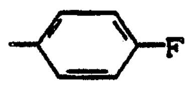 | 2 | 110 |
| 81 | $-CO-CH_3$ | 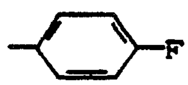 | 2 | 102 |
| 82 | $-CO-CH_3$ | 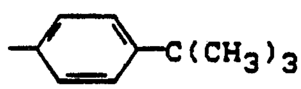 | 2 | 70 |
| 83 | H | 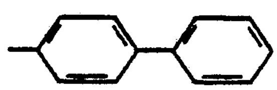 | 1 | 195 |
| 84 | H | 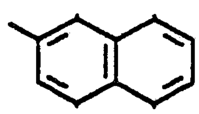 | 1 | 200 |
| 85 | $-CO-CH_3$ | 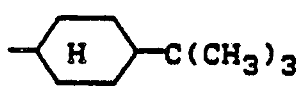 | 0 | 50 |

<u>Tabelle 1</u> - Fortsetzung

| Bsp.-Nr. | $R^1$ | $R^2$ | n | Schmelzpunkt (°C) |
|---|---|---|---|---|
| 86 | $-CO-CH_3$ | 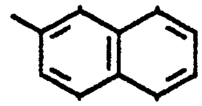 | 1 | 102 |
| 87 | $-CO-CH_3$ | 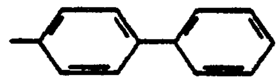 | 1 | 110 |
| 88 | H | 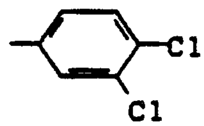 | 1 | 195 |
| 89 | H | 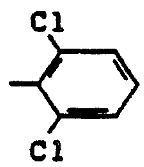 | 1 | 145 |
| 90 | H | 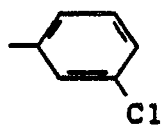 | 1 | 145 |
| 91 | H | 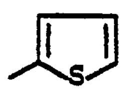 | 1 | 138 |
| 92 | $-CO-CH_3$ | 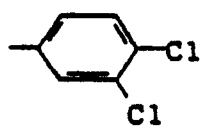 | 1 | 147 |
| 93 | $-CO-CH_3$ | 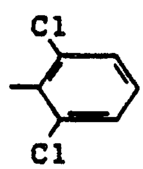 | 1 | 102 |

Tabelle 1 - Fortsetzung

| Bsp.-Nr. | $R^1$ | $R^2$ | n | Schmelzpunkt (°C) |
|---|---|---|---|---|
| 94 | $-CO-CH_3$ | 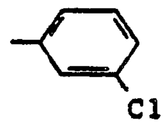 | 1 | 83 |
| 95 | H | 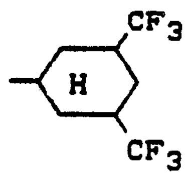 | 0 | 68 |
| 96 | $-CO-CH_3$ | 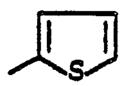 | 1 | 74 |
| 97 | $-CO-CH_3$ | 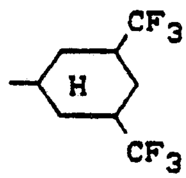 | 0 | 125 |
| 98 | $-CO-CH_2CH_2-$ 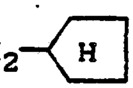 | 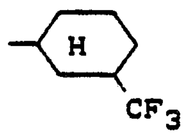 | 1 | 123 |
| 99 | -CO 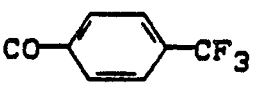 | 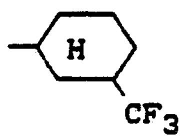 | 1 | |
| 100 | -CO 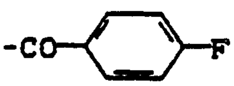 | 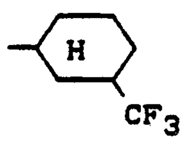 | 1 | 81 |
| 101 | -CO 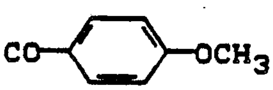 | 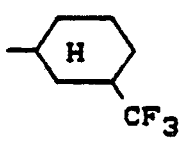 | 1 | |

Tabelle 1 - Fortsetzung

| Bsp.-Nr. | $R^1$ | $R^2$ | n | Schmelzpunkt (°C) |
|---|---|---|---|---|
| 102 | 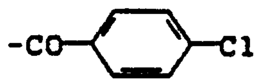 | 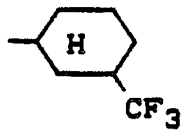 | 1 | 84 |
| 103 | $-CO-CH_3$ | 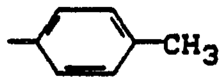 | 0 | 130 |
| 104 | 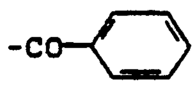 | 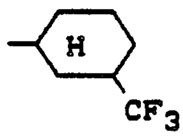 | 1 | 68 |
| 105 | 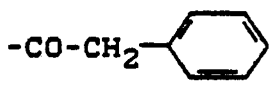 | 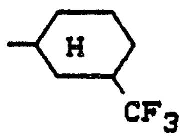 | 1 | 87 |
| 106 | 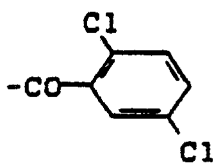 | 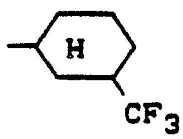 | 1 | 86 |
| 107 | 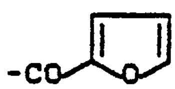 | 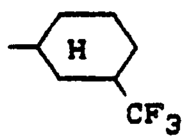 | 1 | |
| 108 | 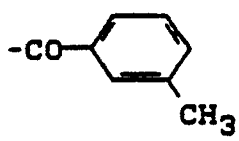 | 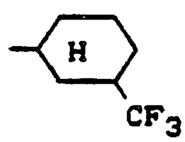 | 1 | 103 |
| 109 | 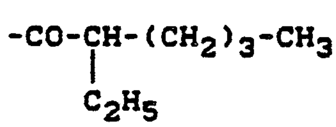 | 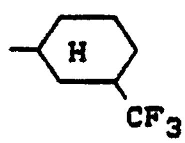 | 1 | 55 |

Tabelle 1 - Fortsetzung

| Bsp.-Nr. | $R^1$ | $R^2$ | n | Schmelzpunkt (° C) |
|---|---|---|---|---|
| 110 | $-CO-(CH_2)_{12}-CH_3$ | 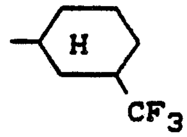 | 1 | 50 |
| 111 | -CO 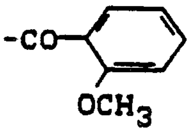 | 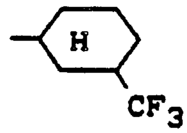 | 1 | 102 |
| 112 | -CO 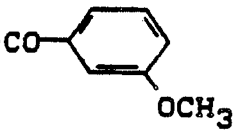 | 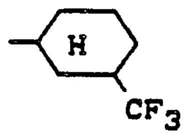 | 1 | 111 |
| 113 | -CO 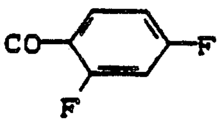 | 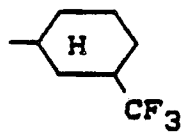 | 1 | 76 |
| 114 | -CO 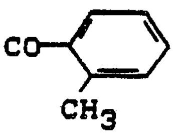 | 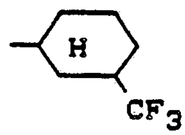 | 1 | 86 |
| 115 | -CO 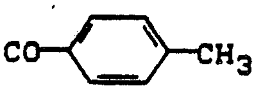 | 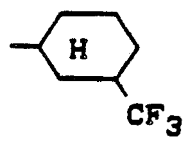 | 1 | 98 |
| 116 | -CO 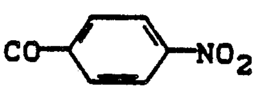 | 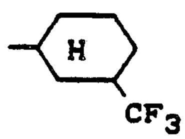 | 1 | 119 |
| 117 | $-CO-C(CH_3)_3$ | 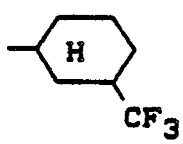 | 1 | 64 |

Tabelle 1 - Fortsetzung

| Bsp.-Nr. | $R^1$ | $R^2$ | n | Schmelzpunkt (°C) |
|---|---|---|---|---|
| 118 | 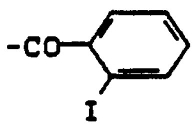 | 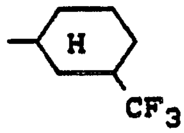 | 1 | 67 |
| 119 | 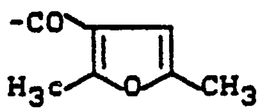 | 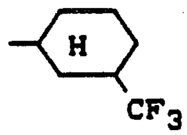 | 1 | 98 |
| 120 | $-CO-CH_3$ | $CF_3$ | 7 | 38 |
| 121 | $-CO-C_2H_5$ | 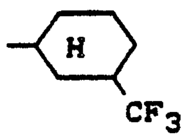 | 1 | 102 |
| 122 | $-CO-(CH_2)_3-CH_3$ | 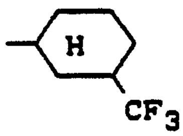 | 1 | |
| 123 | $-CO-(CH_2)_5-CH_3$ | 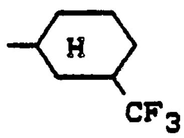 | 1 | |
| 124 | 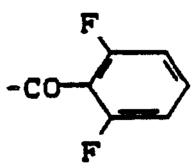 | 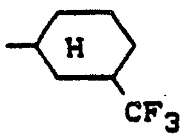 | 1 | 118 |

Die Verbindungen der Formel (I) sind bekannt und/oder können nach an sich bekannten Verfahren hergestellt werden (vgl. Liebigs Ann. Chem. 763 (1972), 135 - 147; Acta Chem. Scand. 27 (1973), 6; J. Am. Chem. Soc. 58 (1936), 1163 - 1167; ibid. 70 (1948), 3165 - 3173; ibid. 70 (1948), 3174 - 3175; ibid. 74 (1952), 3910 - 3915; DE-OS 26 41 343; EP-A 2 228; EP-A 77 550; EP-A 77 551; EP-A 123 238; EP-A 123 239; GB-PS 1 553 424; US 2 553 648; US 3 367 830 und US 3 347 742).

Gegenstand der vorliegenden Erfindung sind weiter neue substituierte 1,4-Naphthochinone der allgemeinen Formel (Ia)

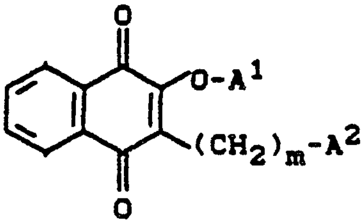 (Ia)

in welcher

m für die Zahlen 0 oder 1 bis 12 steht,
$A^1$ für Wasserstoff, Methyl, Benzyl, Acetyl, Methoxycarbonyl, Benzolsulfonyl oder Toluolsulfonyl steht und
$A^2$ für Perfluor-$C_1$-$C_6$-Alkyl, 2-Trifluormethyl- oder 3-Trifluormethyl-cyclohexyl, 2-Trifluormethyl- oder 3-Trifluormethyl-phenyl, 4-Trimethylsilyl-cyclohexyl oder 4-Trifluormethylthio-phenyl steht
- mit der Maßgabe, daß m von 0 und 2 verschieden ist, wenn $A^2$ für 3-Trifluormethyl-cyclohexyl oder 3-Trifluormethyl-phenyl steht.

Man erhält die neuen Verbindungen der Formel (Ia), wenn man

(a) 2-substituierte 3-Halogen-1,4-naphthochinone der allgemeinen Formel (II)

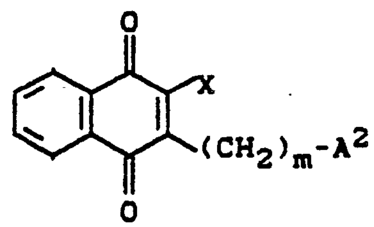

(II)

in welcher
m und $A^2$ die oben angegebenen Bedeutungen haben und
X für Halogen steht,
mit einem Alkalimetall- bzw. Erdalkalimetallhydroxid, gegebenenfalls in Gegenwart eines Verdünnungsmittels umsetzt, oder

(b) 2,3-Epoxy-2,3-dihydro-1,4-naphthochinone der allgemeinen Formel (III)

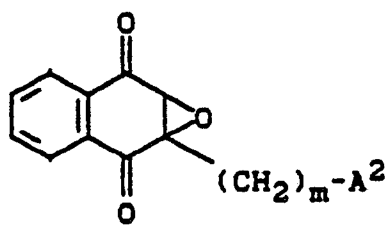

(III)

in welcher
m und $A^2$ die oben angegebenen Bedeutungen haben,
mit einem Alkalimetall- bzw. Erdalkalimetallhydroxid, gegebenenfalls in Gegenwart eines Verdünnungsmittel umsetzt, oder

(c) 2-substituierte 3-Hydroxy-1,4-naphthochinone der allgemeinen Formel (Ib)

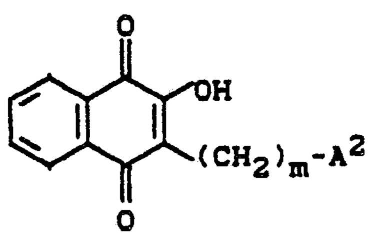

(Ib)

in welcher
m und $A^2$ die oben angegebenen Bedeutungen haben,
mit Verbindungen der allgemeinen Formel (IV)

Y - A

in welcher
A mit Ausnahme von Wasserstoff die oben für $A^1$ angegebene Bedeutung hat und
Y für eine nucleofuge Abgangsgruppe steht,
gegebenenfalls in Gegenwart eines Metallsalzes, gegebenenfalls in Gegenwart eines Säureakzeptors und

gegebenenfalls in Gegenwart eines Verdünnungsmittels umsetzt.

Bevorzugt werden die neuen substituierten 1,4-Naphthochinone der Formel (Ia), in welcher
m für die Zahlen Null oder 1 bis 10 steht,
$A^1$ für Wasserstoff, Methyl, Acetyl oder Benzyl steht und
$A^2$ für Perfluor-$C_1$-$C_6$-Alkyl, 2-Trifluormethyl- oder 3-Trifluormethyl-cyclohexyl, 2-Trifluormethyl- oder 3-Trifluormethyl-phenyl, 4-Trifluormethylsilyl-cyclohexyl oder 4-Trifluormethylthio-phenyl steht
- mit der Maßgabe, daß m von Null und 2 verschieden ist, wenn $A^2$ für 3-Trifluormethyl-cyclohexyl oder 3-Trifluormethyl-phenyl steht.

Verwendet man als Ausgangsstoffe bei der erfindungsgemäßen Verfahrensvariante (a) zur Herstellung der neuen Verbindungen der Formel (Ia) beispielsweise 3-Brom-2-(2- (3-trifluormethyl-phenyl)-ethyl)-1,4-naphthochinon und Kaliumhydroxid, so kann der Reaktionsverlauf durch folgendes Formelschema skizziert werden:

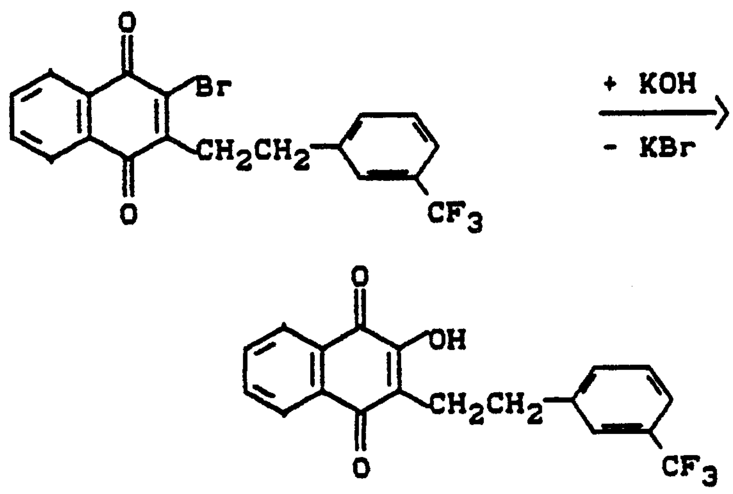


Verwendet man als Ausgangsstoffe bei der erfindungsgemäßen Verfahrensvariante (b) beispielsweise 2,3-Epoxy-2,3-dihydro-2-(4-trimethylsilyl-cyclohexyl-methyl)-1,4-naphthochinon und Natriumhydroxid, so kann der Reaktionsverlauf durch folgendes Formelschema skizziert werden:

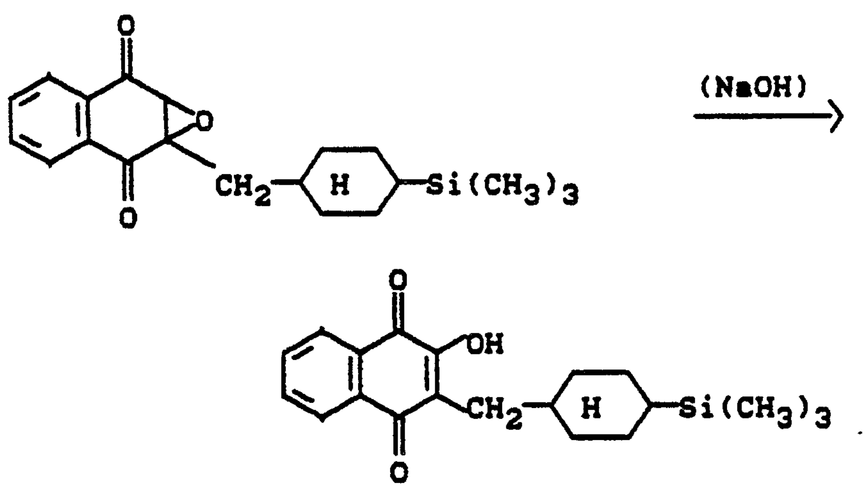


Verwendet man als Ausgangsstoffe bei der erfindungsgemäßen Verfahrensvariante (c) beispielsweise 3-Hydroxy-2-(2,2,2-trifluor-ethyl)-1,4-naphthochinon und Methylbromid, so kann der Reaktionsverlauf durch folgendes Formelschema skizziert werden:

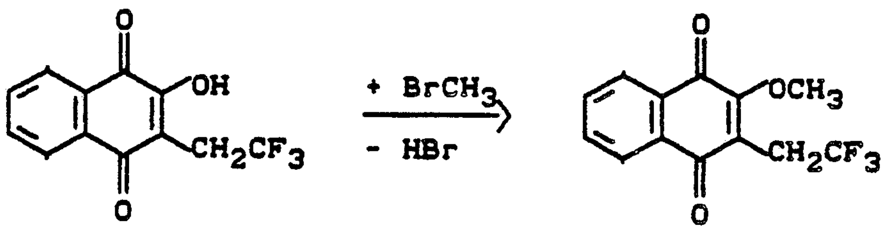


Die bei der Verfahrensvariante (a) als Ausgangstoffe zu verwendenden 2-substituierten 3-Halogen-1,4-naphthochinone sind durch die Formel (II) allgemein definiert. In Formel (II) haben m und $A^2$ vorzugsweise die gleichen Bedeutungen, wie sie oben bei der Beschreibung der neuen Wirkstoffe der Formel (Ia) für m bzw. $A^2$ als bevorzugt angegeben sind und X steht vorzugsweise für Chlor oder Brom.

Beispiele für die neuen Ausgangsstoffe der Formel (II) sind in der nachstehenden Tabelle 2 aufgeführt.

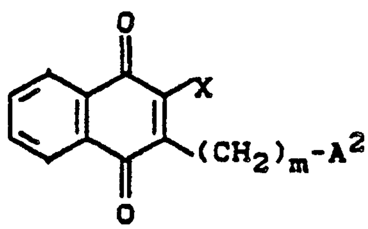

(II)

**Tabelle 2**: Beispiele für Verbindungen der Formel (II)

| Beisp.-Nr. | X | m | $A^2$ | Schmelzpunkt (°C) |
|---|---|---|---|---|
| II-1 | Cl | 1 | 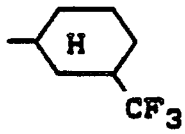 | 104 |
| II-2 | Br | 2 | $CF_3$ | |
| II-3 | Cl | 3 | $CF_3$ | |
| II-4 | Br | 2 | 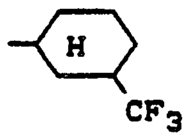 | |
| II-5 | Cl | 1 | 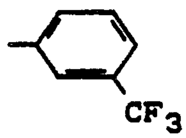 | |
| II-6 | Cl | 0 | 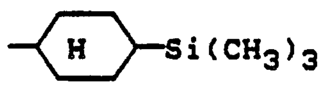 | |
| II-7 | Br | 1 | 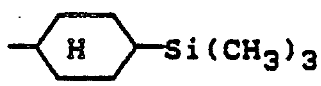 | |
| II-8 | Cl | 0 | 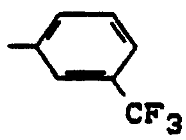 | |
| II-9 | Cl | 0 | 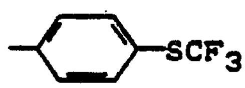 | |
| II-10 | Cl | 1 | 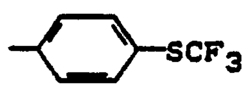 | |
| II-11 | Cl | 2 | 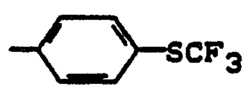 | |

**Tabelle 2** - Fortsetzung

| Beisp.-Nr. | X | m | $A^2$ | Schmelzpunkt (°C) |
|---|---|---|---|---|
| II-12 | Cl | 1 | 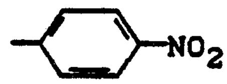 | 158 |
| II-13 | Cl | 1 | 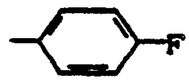 | 115 |
| II-14 | Cl | 1 | 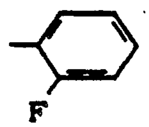 | 150 |
| II-15 | Cl | 1 | 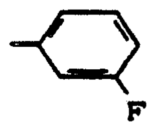 | 132 |
| II-16 | Cl | 1 | 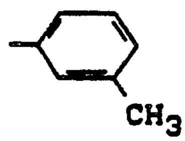 | 138 |
| II-17 | Cl | 1 | 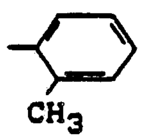 | 140 |
| II-18 | Cl | 1 | 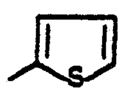 | 103 |

Die Ausgangsstoffe der Formel (II) sind noch nicht aus der Literatur bekannt. Man erhält die neuen 2-substituierten 3-Halogen-1,4-naphthochinone der Formel (II), wenn man 2-Halogen-1,4-naphthochinone der Formel (V)

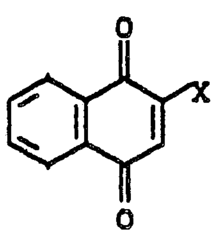

(V)

in welcher

X die oben angegebene Bedeutung hat,

mit Carbonsäuren der Formel (VI)

$A^2 - (CH_2)_m - COOH$ (VI)

in welcher

m und $A^2$ die oben angegebenen Bedeutungen haben
in Gegenwart von Silbernitrat und Ammoniumperoxodisulfat sowie in Gegenwart eines Verdünnungsmittels, wie z. B. Acetonitril, Wasser und Sulfolan, bei Temperaturen zwischen 0 °C und 100 °C, vorzugsweise zwischen 10 °C und 80 °C, umsetzt.

Die als Zwischenprodukte zu verwendenden 2-Halogen-1,4-naphthochinone sind durch die Formel (V) allgemein defi niert. In Formel (V) steht vorzugsweise X für Chlor oder Brom.

Als Beispiele für die Verbindungen der Formel (V) seien genannt:
2-Chlor-1,4-naphthochinon und 2-Brom-1,4-naphthochinon.

Die Verbindungen der Formel (V) sind bekannt (vgl. J. Chem. Soc. 1935, 1850 - 1854).

Die weiter als Zwischenprodukte zu verwendenden Carbonsäuren sind durch die Formel (VI) allgemein definiert. in Formel (VI) haben m und $A^2$ vorzugsweise die gleichen Bedeutungen, wie sie oben bei der Beschreibung der neuen Wirkstoffe der Formel (Ia) für m bzw. $A^2$ als bevorzugt angegeben sind.

Als Beispiele für die Verbindungen der Formel (VI) seien genannt:
3-Trifluormethyl-benzoesäure, (3-Trifluormethyl-phenyl)-essigsäure, ω-(3-Trifluormethyl-phenyl)-propionsäure, 3-Trifluormethyl-cyclohexan-carbonsäure, (3-Trifluormethyl-cyclohexyl)-essigsäure, ω-(3-Tri fluormethyl-cyclohexyl)-propionsäure, 4-Trimethylsilyl-cyclohexan-carbonsäure, (4-Trimethylsilyl-cyclohexyl)-essigsäure, ω(4-Trimethylsilyl-cyclohexyl)-propionsäure, 4-Trifluormethylthio-benzoesäure, (4-Trifluormethylthio-phenyl)-essigsäure und ω-(4-Trifluormethyl-phenyl)-propionsäure.

Die Verbindungen der Formel (VI) sind bekannt und/oder können nach an sich bekannten Verfahren hergestellt werden (vgl. EP-A 2 228, EP-A 77 550, EP-A 77 551, EP-A 123 238). Beispielsweise erhält man die Verbindungen der Formel (VI) in welcher $A^2$ für 3-Trifluormethyl-cyclohexyl bzw. 4-Trimethylsilyl-cyclohexyl steht, wenn man die entsprechenden Verbindungen der Formel (VI) in welcher $A^2$ für 3-Trifluormethyl-phenyl bzw. 4-Trimethylsilyl-phenyl steht, nach üblichen Methoden, beispielsweise in Gegenwart von Rhodium- oder Ruthenium-Katalysatoren, hydriert (vgl. P. N. Rylander, Catalytic Hydrogenation in Organic Syntheses, Academic-Press, New-York 1979).

Die bei der Verfahrensvariante (b) als Ausgangsstoffe zu verwendenden 2,3-Epoxy-2,3-dihydro-1,4-naphthochinone sind durch die Formel (III) allgemein definiert. In Formel (III) haben m und $A^2$ vorzugsweise die gleichen Bedeutungen, wie sie oben bei der Beschreibung der neuen Wirkstoffe der Formel (Ia) für m bzw. $A^2$ als bevorzugt angegeben sind.

Beispiele für die neuen Ausgangsstoffe der Formel (III) sind in der nachstehenden Tabelle 3 aufgeführt.

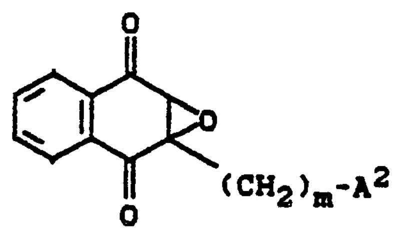

(III)

<u>Tabelle 3</u>: Beispiele für Verbindungen der Formel (III)

| Beispiel-Nr. | m | $A^2$ | Schmelzpunkt (°C) |
|---|---|---|---|
| III-1 | 1 | 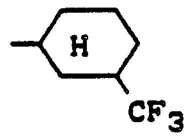 | 85 |
| III-2 | 2 | 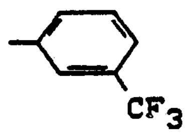 | |
| III-3 | 2 | $CF_3$ | |
| III-4 | 3 | $CF_3$ | |
| III-5 | 2 | 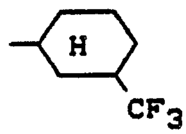 | |
| III-6 | 1 | 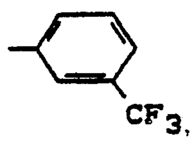 | |
| III-7 | 0 | 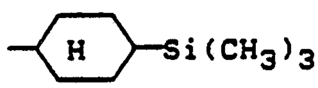 | |
| III-8 | 1 | 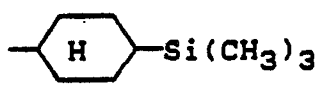 | |
| III-9 | 0 | 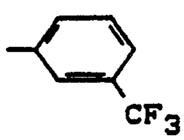 | |
| III-10 | 0 | 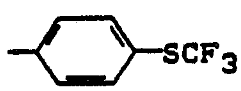 | |

Tabelle 3 - Fortsetzung

| Beispiel-Nr. | m | $A^2$ | Schmelzpunkt (° C) |
|---|---|---|---|
| III-11 | 1 | 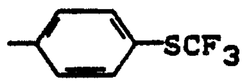 | |
| III-12 | 2 | 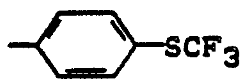 | |

Die Ausgangsstoffe der Formel (III) sind noch nicht aus der Literatur bekannt. Man erhält die neuen 2,3-Epoxy-2,3-dihydro-1,4-naphthochinone der Formel (II), wenn man 2-substituierte 1,4-Naphthochinone der Formel (VII)

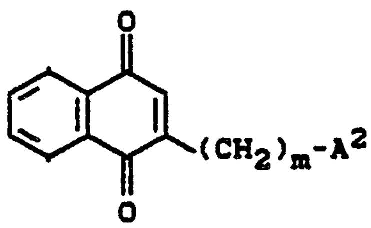

(VII)

in welcher
m und $A^2$ die oben angegebenen Bedeutungen haben,
mit Hydrogenperoxid in Gegenwart eines Verdünnungsmittels, wie z. B. Wasser und Dioxan, und gegebenenfalls in Gegenwart eines Säureakzeptors, wie z. B. Natrium-oder Kalium-carbonat, bei Temperaturen zwischen 0 °C und 100 °C, vorzugsweise zwischen 10 °C und 80 °C umsetzt.

Die als Zwischenprodukte zu verwendenden 2-substituierten 1,4-Naphthochinone sind durch die Formel (VII) allgemein definiert.

In Formel (VII) haben m und $A^2$ vorzugsweise die gleichen Bedeutungen, wie sie oben bei der Beschreibung der neuen Wirkstoffe der Formel (Ia) für m bzw. $A^2$ als bevorzugt angegeben sind.

Beispiele für die neuen Zwischenproukte der Formel (VII) sind in der nachstehenden Tabelle 4 aufgeführt.

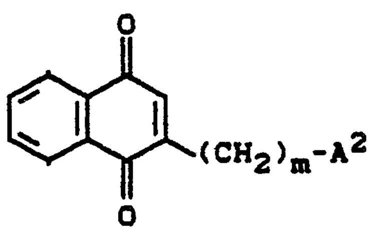

(VII)

**Tabelle 4**: Beispiele für Verbindungen der Formel (VII)

| Beispiel-Nr. | m | $A^2$ | Schmelzpunkt (°C) |
|---|---|---|---|
| VII-1 | 1 | 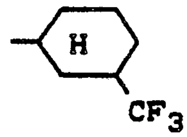 | 65 |
| VII-2 | 2 | 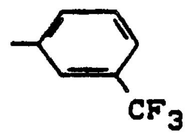 | |
| VII-3 | 2 | $CF_3$ | |
| VII-4 | 3 | $CF_3$ | |
| VII-5 | 2 | 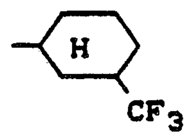 | |
| VII-6 | 1 | 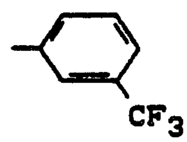 | |
| VII-7 | 0 | 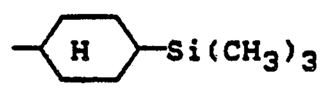 | |

Tabelle 4 - Fortsetzung

| Beispiel-Nr. | m | $A^2$ | Schmelzpunkt (°C) |
|---|---|---|---|
| VII-8 | 1 | 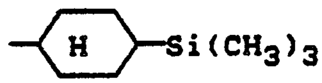 | |
| VII-9 | 0 | 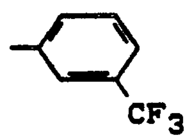 | |
| VII-10 | 0 | 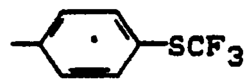 | |
| VII-11 | 1 | 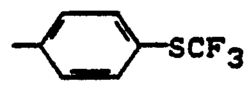 | |
| VII-12 | 2 | 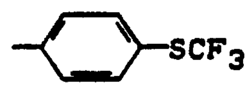 | |

Die Zwischenprodukte der Formel (VII) sind noch nicht aus der Literatur bekannt. Man erhält die neuen 2-substituierten 1,4-Naphthochinone der Formel (VII), wenn man 1,4-Naphthochinon mit Carbonsäuren der Formel (VI) - oben - in Gegenwart von Silbernitrat und Ammoniumperoxodisulfat sowie in Gegenwart eines Verdünnungsmittels, wie z. B. Acetonitril und Wasser, bei Temperaturen zwischen 0 °C und 100 °C, vorzugsweise zwischen 10 °C und 80 °C, umsetzt.

Bezüglich der Carbonsäuren der Formel (VI) sei auf die obigen Angaben verwiesen.

Die bei der Verfahrensvariante (c) als Ausgangsstoffe zu verwendenden 2-substituierten 3-Hydroxy-1,4-naphthochinone sind durch die Formel (Ib) allgemein definiert. In Formel (Ib) haben m und $A^2$ vorzugsweise die gleichen Bedeutungen, wie sie oben bei der Beschreibung der neuen Wirkstoffe der Formel (Ia) für m bzw. $A^2$ als bevorzugt angegeben sind.

Beispiele für Verbindungen der Formel (Ib) sind Tabelle 1 (Beispiel 2, 5, 6, 7, 8) zu entnehmen.

Die 2-substituierten 3-Hydroxy-1,4-naphthochinone der Formel (Ib) sind neue, erfindungsgemäße Verbindungen, die nach den Verfahrensvarianten (a) und (b) hergestellt werden können.

Die bei der Verfahrensvariante (c) weiter als Ausgangsstoffe zu verwendenden Verbindungen sind durch die Formel (IV) allgemein definiert. Als nucleofuge Abgangsgruppe Y steht in Formel (IV) vorzugsweise Halogen. In Formel (IV) stehen insbesondere

$A^1$ für Methyl oder Acetyl und

Y für Chlor, Brom oder Iod.

Als Beispiele für die Verbindungen der Formel (IV) seien genannt:

Methylchlorid, Methylbromid, Methyliodid und Acetylchlorid.

Die Verbindungen der Formel (IV) sind bekannte Chemikalien.

Die erfindungsgemäße Verfahrensvariante (a) wird vorzugsweise in Gegenwart eines Verdünnungsmittels durchgeführt. Als solche kommen insbesondere polare Lösungsmittel, wie z. B. Wasser, Methanol, Ethanol, n- und iso-Propanol, Ethylenglycol sowie dessen Monomethylether und Dimethylether, Dioxan, Dimethylsulfoxid und Dimethylformamid in Betracht.

Gemische aus Wasser und Methanol werden besonders bevorzugt eingesetzt.

Für das erfindungsgemäße Verfahren - Variante (a) -werden Alkalimetall- bzw. Erdalkalimetall-hydroxide eingesetzt. Beispiele hierfür sind Lithium-, Natrium-, Kalium-, Magensium- und Calciumhydroxid.

Natriumhydroxid und Kaliumhydroxid werden besonders bevorzugt eingesetzt.

Die Reaktionstemperaturen können bei dem erfindungsgemäßen Verfahren (a) in einem größeren

Bereich variiert werden. Im allgemeinen arbeitet man bei Temperaturen zwischen 0 °C und 150 °C, vorzugsweise bei Temperaturen zwischen 20 °C und 100 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung der erfindungsgemäßen Verfahrensvariante (a) setzt man je Mol Ausgangsverbindung der Formel (II) im allgemeinen zwischen 1 und 3 Mol, vorzugsweise zwischen 1 und 2 Mol, Alkalimetall- bzw. Erdalkalimetall-hydroxid ein. Die Reaktionskomponenten werden im allgemeinen bei Raumtemperatur zusammengegeben und bei etwas erhöhter Temperatur bis zum Ende der Umsetzung gerührt.

Die Aufarbeitung kann nach üblichen Methoden durchgeführt werden: beispielsweise, indem man mit einer verdünnten wäßrigen Säure, wie z. B. Salzsäure, ansäuert, das Produkt durch Absaugen isoliert und gegebenenfalls durch Umkristallisation reinigt.

Für die erfindungsgemäße Verfahrensvariante (b) kommen vorzugsweise die gleichen Verdünnungsmittel zum Einsatz, wie sie oben für Verfahren (a) angegeben sind. Gemische aus Wasser und Dimethylsulfoxid werden besonders bevorzugt.

Ebenso können für Verfahren (b) die gleichen Alkalimetall- bzw. Erdalkalimetall-hydroxide eingesetzt werden, wie sie oben für Verfahren (a) angegeben sind. Natriumhydroxid und Kaliumhydroxid werden ebenfalls besonders bevorzugt.

Die Reaktionstemperaturen können bei dem erfindungsgemäßen Verfahren (b) in einem größeren Bereich variiert werden. Im allgemeinen arbeitet man bei Temperaturen zwischen 0 °C und 150 °C, vorzugsweise bei Temperaturen zwischen 20 °C und 120 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung der erfindungsgemäßen Verfahrensvariante (b) setzt man je Mol Ausgangsverbindung der Formel (III) im allgemeinen zwischen 1 und 5 Mol, vorzugsweise zwischen 1 und 3 Mol, Alkalimetall- bzw. Erdalkalimetall-hydroxid ein. Die Umsetzung und Aufarbeitung wird im allgemeinen wie oben für Verfahren (a) angegeben durchgeführt.

Das erfindungsgemäße Verfahren (c) zur Herstellung der neuen Verbindungen der Formel (Ia) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenen falls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlrobenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und-ethylester, Nitrile wie z. B. Acetonitril und Propionitril, Amide wie z. B. Dimethylformamid, Dimethylacetamid und N-Methyl-pyrrolidon sowie Diemthylsulfoxid, Tetramethylensulfon und Hexamethylphosphorsäuretriamid.

Als Säureakzeptoren können bei dem erfindungsgemäßen Verfahren (c) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die erfindungsgemäße Verfahrensvariante (c) wird gegebenenfalls in Gegenwart eines Metallsalzes durchgeführt. Als solche kommen insbesondere Schwermetallsalze, wie z. B. Kupfersulfat, Kupfernitrat, Silbersulfat, Silbernitrat, Quecksilbersulfat und Quecksilbernitrat, in Betracht.
Silbersalze, wie z. B. Silbernitrat, werden besonders bevorzugt.

Die Reaktionstemperaturen können bei dem erfindungsgemäßen Verfahren (c) in einem größeren Bereich variiert werden. Im allgemeinen arbeitet man bei Temperaturen zwischen 0 °C und 100 °C, vorzugsweise bei Temperaturen zwischen 10 °C und 80 °C.

Zur Durchführung der erfindungsgemäßen Verfahrensvariante (c) werden die Ausgangsstoffe der Formeln (Ib) und (IV) im allgemeinen in angenähert äquimolaren Mengen eingesetzt. Die Verbindungen der Formel (IV) können jedoch auch im Überschuß bis etwa 100 % problemlos eingesetzt werden. Die Reaktionskomponenten werden im allgemeinen bei Raumtemperatur zusammengegeben und gegebenenfalls bei erhöhter Temperatur bis zum Reaktionsende gerührt. Die Aufarbeitung kann nach üblichen Methoden durchgeführt werden. Beispielsweise wird - gegebenenfalls nach Einengen - mit einer wäßrigen Säure, wie z. B. Salzäure, verdünnt und mit einem mit Wasser praktisch nicht mischbaren Lösungsmittel, wie z. B. Methylenchlorid, extrahiert. Die Extraktionslösung wird - gegebenenfalls nach Waschen mit

Wasser - getrocknet, filtriert und eingeengt. Der Rückstand enthält im Wesentlichen das Produkt der Formel (Ia), das nach üblichen Methoden, z. B. durch Umkristallisation, weiter gereinigt werden kann.

Die Wirkstoffe eignen sich zur Bekämpfung von tierischen Schädlingen, vorzugsweise Arthropoden und Nematoden, insbesondere Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixius, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosooma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelatica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymeoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cutrebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio mauraus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidognye spp., Aphelenchoides spp., Longidorus spp., Xiphinema spp., Trichodorus spp..

Die erfindungsgemäßen Wirkstoffe zeigen insbesondere hervorragende akarizide Wirkung, auch gegen resistente Spinnmilben, wie z. B. Tetranychus urticae, welche Nutzpflanzen, wie z. B. Bohnen schädigen.

Die erfindungsgemäßen Wirkstoffe weisen eine stark mikrobizide Wirkung auf und können zur Bekämpfung von unerwünschten Mikroorganismen praktisch eingesetzt werden. Die Wirkstoffe sind für den Gebrauch als Pflanzenschutzmittel eingesetzt.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten wie beispielsweise Venturia inaequalis;
Pyrenophora-Arten, wie beispielsweise Pyrenophora teres oder P. graminae
(Konidienform: Drechslera, Syn: Helminthosporium);
Cochlibolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptoshphaeria nodorum;
Cercospora-Arten, wie beispielsweise Cercospora canescens;
Altenaria-Arten, wie beispielsweise Alternaria brassicae;
Pseudocercosoporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe zeigen insbesondere starke fungizide Wirkung, z. B. protektive Wirkung gegen den Apfelschorferreger (Venturia inaequalis).

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als

Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfonate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäßen Wirkstoffe geschieht auf diesem Gebiet in bekannter Weise, die durch äußerliche Anwendung in Form beispielsweise des Tauchens (Dippen), Sprühens (Sprayen), Aufgießen (pour-on and spot-on) und des Einpuderns.

Die biologische Wirksamkeit der erfindungsgemäßen Verbindungen soll anhand der folgenden Beispiele erläutert werden.

Anwendungsbeispiele

In den folgenden Anwendungsbeispielen werden die nachstehend aufgeführten Verbindungen als Vergleichssubstanzen eingesetzt:

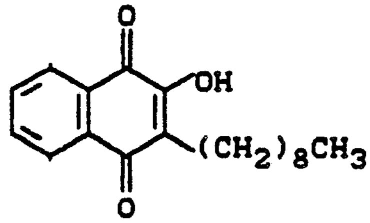

(A)

2-Nonyl-3-hydroxy-1,4-naphthochinon

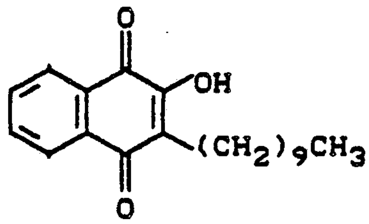

(B)

2-Decyl-3-hydroxy-1,4-naphthochinon

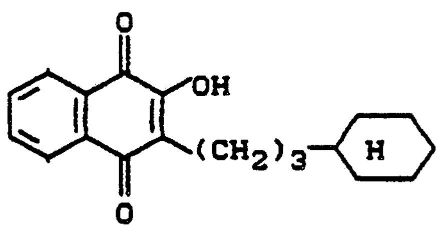

(C)

2-(3-Cyclohexyl-propyl)-3-hydroxy-1,4-naphthochinon
(alle bekannt US-PS 2 572 946.

Beispiel A

Tetranychus-Test (resistent)

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulagaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

In diesem Test zeigen z. B. die in Tabelle 1 als Beispiele Nr. (1), (2), (3), (4), (5) und (9) aufgeführten erfindungsgemäßen Verbindungen erheblich stärkere Wirkung als die Vergleichssubstanz (B).

**Tabelle A**

(pflanzenschädigende Milben)
Tetranychus-Test

| Wirkstoff | Wirkstoffkonzentration in % | Abtötungsgrad in % nach 7 d |
|---|---|---|
| 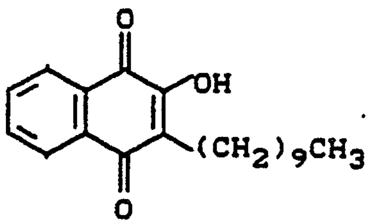 (B) (bekannt) | 0,01 | 0 |
| 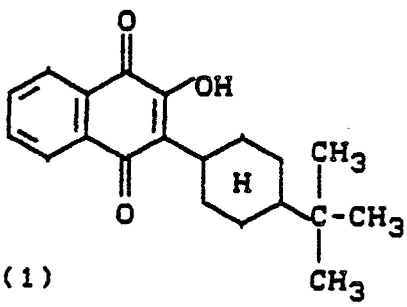 (1) | 0,01 | 90 |
| 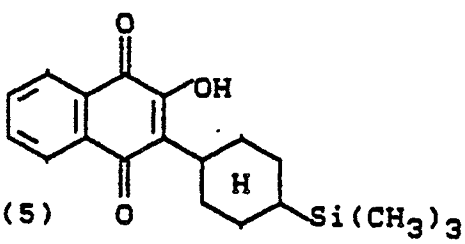 (5) | 0,01 | 65 |
| 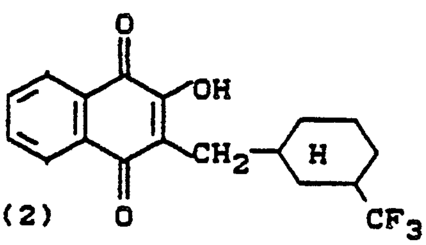 (2) | 0,01 | 98 |

## Tabelle A

(pflanzenschädigende Milben)
Tetranychus-Test

| Wirkstoff | Wirkstoffkonzentration in % | Abtötungsgrad in % nach 7 d |
|---|---|---|
| 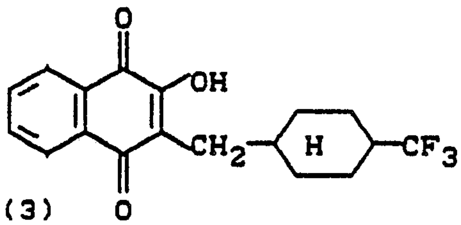 (3) | 0,01 | 100 |
| 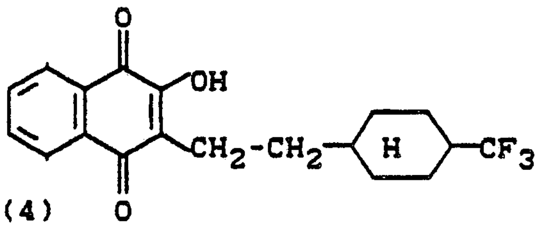 (4) | 0,01 | 100 |
| 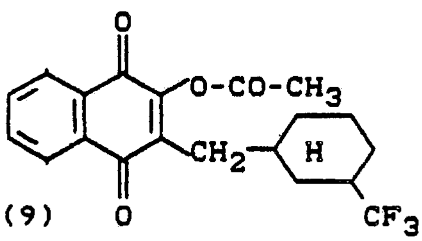 (9) | 0,01 | 100 |

Beispiel B

Eisterilitäts- und Entwicklungshemmtest mit Tetranychus urticae (Gemeine Spinnmilbe)

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Die Blätter einer Bohnenpflanze (Phaseolus vulgaris) taucht man in die entsprechend konzentrierte Wirkstoffzubereitung. Nach Antrocknen der Wirkstoffzubereitung besetzt man die Blätter für ca. 16 Stunden mit Spinnmilben-Weibchen zur Eiablage (ca. 50 Eier/Wiederholung). Die Summe der sterilen und/oder abgetöteten Eier und der abgetöteten Larven, Nymphen und Ruhestadien einer Generation bezogen auf die Zahl der abgelegten Eier ergibt die Abtötung in %. Dabei bedeutet 100 %, daß alle Tiere abgetötet wurden; 0 % bedeutet, daß keine Tiere abgetötet wurden.

Bei diesem Test zeigen z. B. die in Tabelle 1 als Beispiele Nr. (6), (7) und (9) aufgeführten erfindungsgemäßen Verbindungen erheblich stärkere Wirkung als die Vergleichsverbindungen (A), (B) und (C).

## Tabelle B

(pflanzenschädigende Milben)
Tetranychus-Test

| Wirkstoff | Wirkstoffkonzentration in % | Abtötungsgrad in % nach 14 d |
|---|---|---|
| 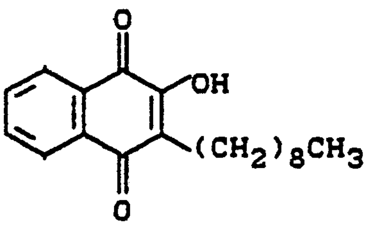 (A) (bekannt) | 0,01 | 0 |
| 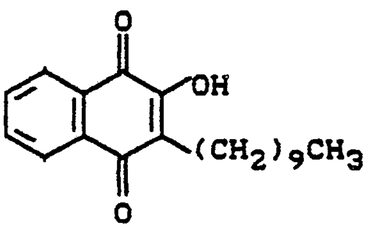 (B) (bekannt) | 0,01 | 0 |
| 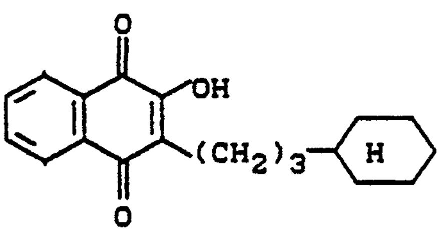 (C) (bekannt) | 0,01 | 40 |
| (9) 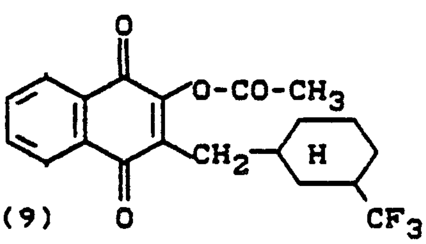 | 0,01 | 100 |

## Tabelle B

(pflanzenschädigende Milben)
Tetranychus-Test

| Wirkstoff | Wirkstoffkonzentration in % | Abtötungsgrad in % nach 14 d |
|---|---|---|
| 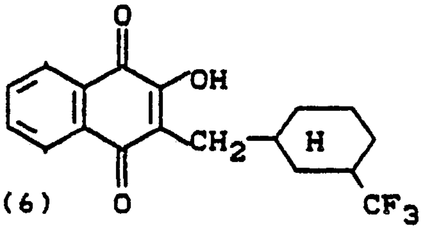 (6) | 0,01 | 100 |
| 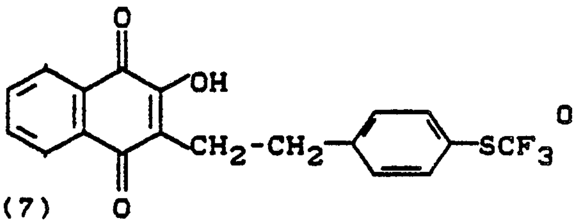 (7) | 0,01 | 100 |

Beispiel C

Venturia-Test (Apfel) /protektiv

Lösungsmittel: 4,7 Gewichtsteile Aceton
Emulgator: 0,3 Gewichtsteile Alkylarylpolyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocken des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z. B. die Verbindungen, welche in Tabelle 1 als Beispiele Nr. (2), (3), (5), (9), (10), (12) (und) (13) aufgeführt sind.

## Tabelle C

Venturia-Test (Apfel)/ protektiv

| Wirkstoff | Befall in % bei einer Wirkstoffkonzentration von 10 ppm |
|---|---|
| 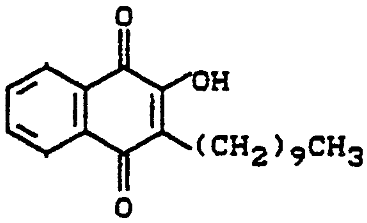 (B) (bekannt) | 44 |
| 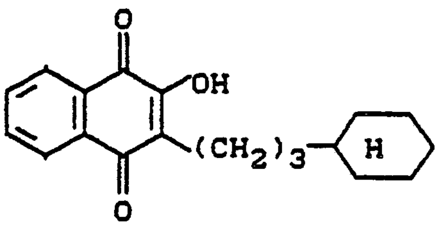 (C) (bekannt) | 17 |
| 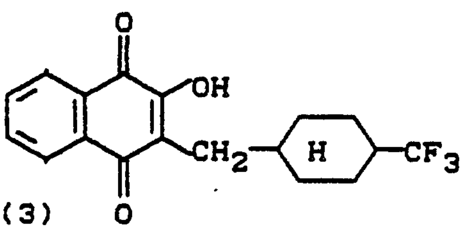 (3) | 0 |
| 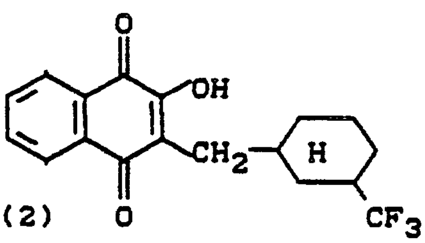 (2) | 0 |
| 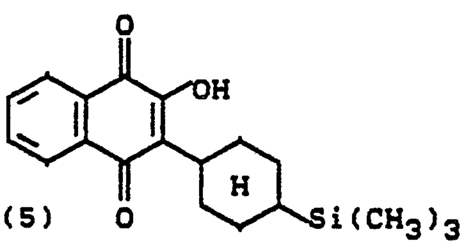 (5) | 3 |

## Tabelle C

### Venturia-Test (Apfel)/ protektiv

| Wirkstoff | Befall in % bei einer Wirkstoffkonzentration von 10 ppm |
|---|---|
| 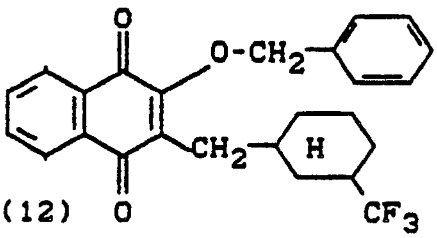 (12) | 15 |
| 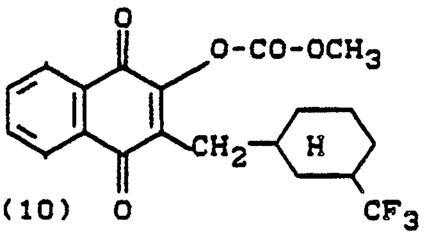 (10) | 7 |
| 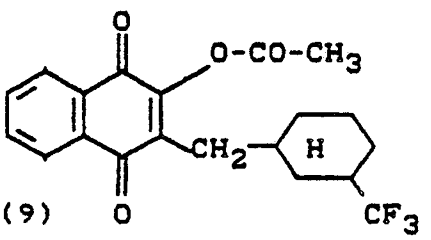 (9) | 0 |
| 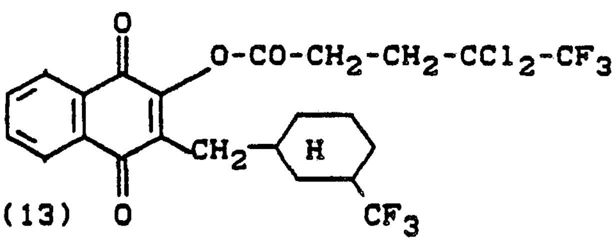 (13) | 4 |

Beispiel D

Test mit Psoroptes ovis

Lösungsmittel: 35 Gewichtsteile Ethylenglykolmonomethylether
35 Gewichtsteile Nonylphenolpolyglykolether
Zur Herstellung einer zweckmäßigen Wirkstoffzubereitung vermischt man drei Gewichtsteile Wirkstoff mit sieben Gewichtsteilen des oben angegebenen Lösunsmittel-Gemisches und verdünnt das so erhaltene Konzentrat mit Wasser auf die gewünschte Konzentration.

Etwa 10 - 25 Psoroptes ovis werden in 1 ml der zu testenden Wirkstoffzubereitung gebracht, die in

Tablettennester einer Tiefziehverpackung pipettiert wurden. Nach 24 Stunden wird der Abtötungsgrad bestimmt.

In diesem Test zeigen z. B. die in Tabelle 1 als Beispiele (1), (2), (3), (4), (5) und (7) aufgeführten Verbindungen sehr starke Wirkung.

## Tabelle D

### Psoroptes ovis-Test

| Wirkstoff | Wirkstoffkonzentration [ppm a.i.] | Abtötende Wirkung in % Psoroptes ovis |
|---|---|---|
| 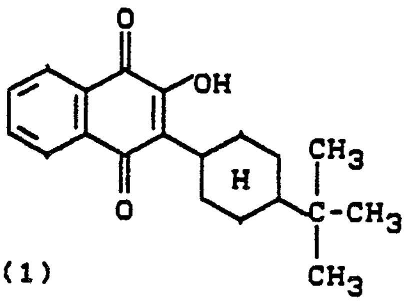 (1) | 10 | 100 |
| | 3 | 100 |
| | 1 | 100 |
| | 0,3 | 100 |
| | 0,1 | 100 |
| | 0,03 | 100 |
| | 0,01 | 100 |
| | 0,003 | 0 |
| 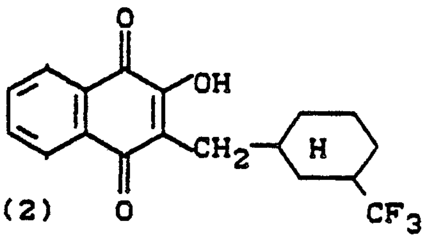 (2) | 10 | 100 |
| | 3 | 100 |
| | 1 | 100 |
| 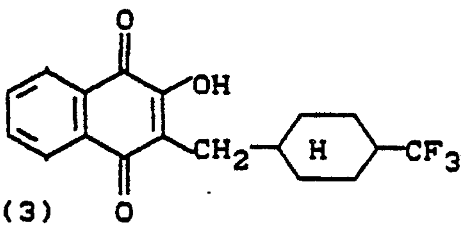 (3) | 10 | 100 |
| | 3 | 100 |
| | 1 | 100 |
| | 0,3 | 0 |
| 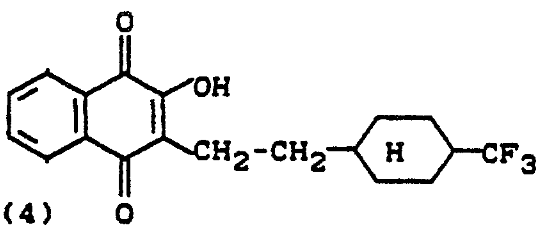 (4) | 10 | 100 |
| | 3 | 100 |
| | 1 | 100 |
| | 0,3 | 100 |
| | 0,1 | 100 |
| | 0,03 | 0 |
| 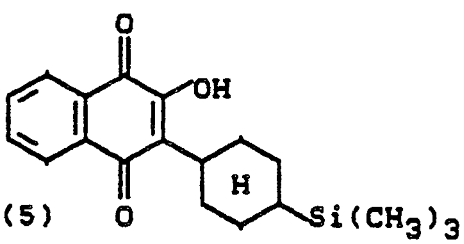 (5) | 10 | 100 |
| | 3 | 100 |
| | 1 | 100 |
| | 0,3 | 100 |
| | 0,1 | 100 |
| | 0,03 | 0 |

## Tabelle D

### Psoroptes ovis-Test

| Wirkstoff | Wirkstoffkonzentration [ppm a.i.] | Abtötende Wirkung in % Psoroptes ovis |
|---|---|---|
| 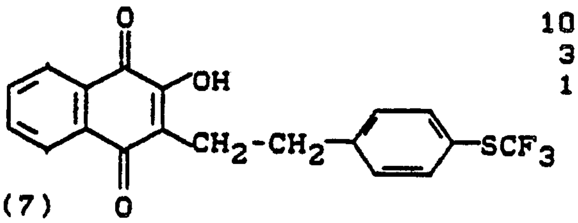 (7) | 10<br>3<br>1 | 100<br>100<br>100 |

Beispiel E

Erysiphe-Test (Weizen) / protektiv

Lösungsmittel: 100 Gewichtsteile Dimethylformamid
Emulgator: 0,25 Gewichtsteile Alkylarylpolyglycolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen in diesem Test z. B. die Verbindungen, welche in Tabelle 1 als Beispiele Nr. (57) und (65) aufgeführt sind.

## Tabelle E

Erysiphe-Test (Weizen) / protektiv

| Wirkstoff | Wirkstoff-konzentration in der Spritzbrühe in Gew.-% | Wirkungsgrad in % der unbehandelten Kontrolle |
|---|---|---|
| 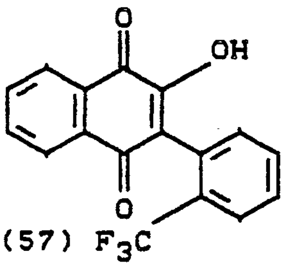 (57) | 0,025 | 100 |
| 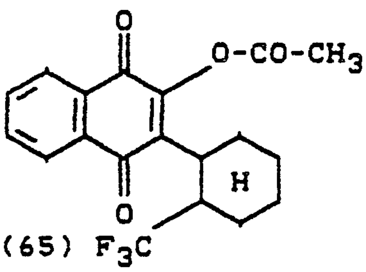 (65) | 0,025 | 100 |

Beispiel F

Erysiphe-Test (Gerste) / protektiv

Lösungsmittel: 100 Gewichtsteile Dimethylformamid
Emulgator: 0,25 Gewichtsteile Alkylarylpolyglycolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenhiet in der Wirksamkeit gegenüber dem Stand der Technik zeigen in diesem Test z. B. die Verbindungen, welche in Tabelle 1 als Beispiele Nr. (57) und (65) aufgeführt sind.

## Tabelle F

### Erysiphe-Test (Gerste) / protektiv

| Wirkstoff | Wirkstoff-konzentration in der Spritzbrühe in Gew.-% | Wirkungsgrad in % der unbehandelten Kontrolle |
|---|---|---|
| 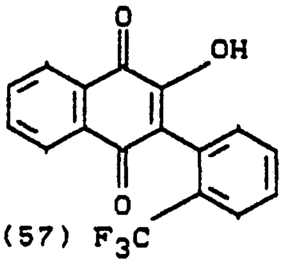 (57) | 0,025 | 100 |
| 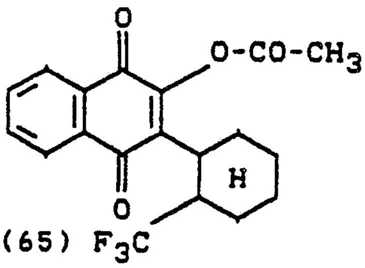 (65) | 0,025 | 100 |

Herstellungsbeispiele

Beispiel 1

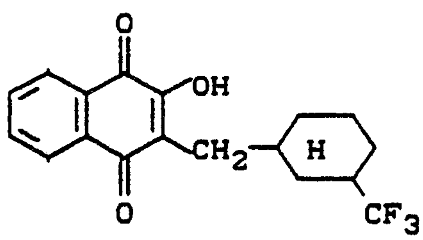

Eine Mischung aus 22 g (0,062 Mol) 3-Chlor-2-(3-trifluormethyl-1-cyclohexyl-methyl)-1,4-naphthochinon, 5,2 g (0,09 Mol) Kaliumhydroxid, 50 ml Methanol und 50 ml Wasser wird 60 Minuten unter Rückfluß zum Sieden erhitzt. Nach dem Abkühlen wird mit 2N-Salzsäure angesäuert, abgesaugt und aus Isopropanol umkristallisiert.

Man erhält 15 g (72 % der Theorie) 2-(3-Trifluormethyl-1-cyclohexyl-methyl)-3-hydroxy-1,4-naphthochinon vom Schmelzpunkt 142 °C.

Beispiel 2

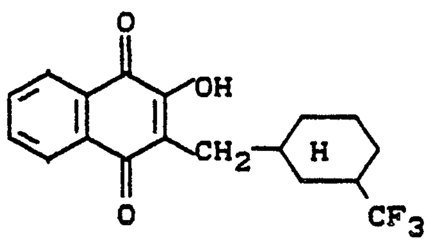

Eine Mischung aus 10 g (0,03 Mol) 2,3-Epoxy-2,3-dihydro-2-(3-trifluormethyl-1-cyclohexylmethyl)-1,4-naphthochi non, 5 g (0,09 Mol) Kaliumhydroxid, 15 ml Wasser und 85 ml Dimethylsulfoxid wird 60 Minuten bei 100 °C gerührt. Nach dem Abkühlen wird mit 2N-Salzsäure neutralisiert und abgesaugt.

Man erhält 8,3 g (83 % der Theorie) 2-(3-Trifluormethyl-1-cyclohexyl-methyl)-3-hydroxy-1,4-naphthochinon vom Schmelzpunkt 142 °C.

Analog zu den Herstellungsbeispielen 1 und 2 - welche zu der als Beispiel 1 in Tabelle 1 aufgeführten Verbindung führen - können auch die in Tabelle 1 als Beispiele Nr. 1, 3, 4, 5, 6, 7 und 8 aufgeführten Verbindungen der Formel (I) hergestellt werden.

Beispiele 3

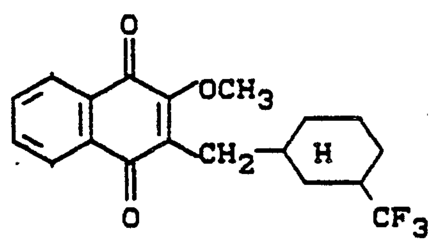

2,8 g (0,02 Mol) Iodmethan werden tropfenweise zu einer Mischung aus 3,4 g (0,01 Mol) 2-(3-Trifluormethyl-1-cyclohexyl-methyl)-3-hydroxy-1,4-naphthochinon, 1,38 g (0,01 Mol) Kaliumcarbonat, 1,69 g (0,01 Mol) Silbernitrat und 50 ml Aceton unter Rühren gegeben und das Reaktionsgemisch wird 6 Stunden unter Rückfluß gerührt. Anschließend wird mit 2N-Salzsäure angesäuert, filtriert und das Filtrat mit Dichlormethan extrahiert. Vom Extrakt wird das Lösungsmittel abdestilliert und der Rückstand wird aus Isopropanol umkristallisiert.

Man erhält 2,8 g (88 % der Theorie) 2-(3-Trifluormethyl-1-cyclohexyl-methyl)-3-methoxy-1,4-naphthochinon vom Schmelzpunkt 90 °C (vgl. Beispiel Nr. 15 in Tabelle 1).

Beispiel 4

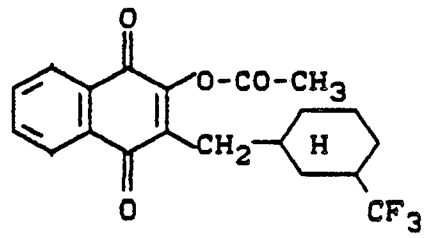

2,0 g (0,026 Mol) Acetylchlorid werden tropfenweise zu einer Mischung aus 8,5 g (0,025 Mol) 2-(3-Trifluormethyl-1-cyclohexyl-methyl)-3-hydroxy-1,4-naphthochinon, 3,0 g (0,03 Mol) Triethylamin und 50 ml Methylenchlorid unter Rühren gegeben und das Reaktionsgemisch wird 2 Stunden bei 20 °C gerührt. Anschließend wird mit 2N-Salzsäure angesäuert, die organische Phase abgetrennt und davon das Lösungsmittel unter vermindertem Druck abdestilliert.

Man erhält 8,74 g (92 % der Theorie) 2-(3-Trifluormethyl-1-cyclohexyl-methyl)-3-acetoxy-1,4-naphtochinon als kristallinen Rückstand vom Schmelzpunkt 110 °C (vgl. Beispiel Nr. 9 in Tabelle 1).

Analog zu den Herstellungsbeispielen 3 bzw. 4 - welche zu den als Beispiel Nr. 15 bzw. 9 in Tabelle 1 aufgeführten Verbindungen führen - können auch die in Tabelle 1 als Beispiele Nr. 10, 11, 12, 13, 14, 16, 17, 18, 19, 20 und 21 aufgeführten Verbindungen der Formel (I) hergestellt werden.

Ausgangsstoffe der Formel (II)

Beispiel (II-1)

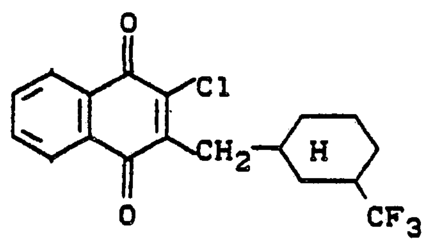

Eine Mischung aus 15 g (0,078 Mol) 2-Chlor-1,4-naphtochinon, 21,3 g (0,1 Mol) (3-Trifluormethyl-cyclohexyl)-essigsäure, 4,5 g (0,027 Mol) Silbernitrat, 100 ml Acetonitril und 50 ml Sulfolan wird auf 65 °C erhitzt. Bei dieser Temperatur wird eine Lösung von 26,3 g (0,155 mol) Ammoniumperoxodisulfat in Wasser innerhalb von 30 Minuten tropfenweise dazu gegeben und das Reaktionsgemisch wird weitere 30 Minuten bei dieser Temperatur gerührt. Dann wird es auf Eis gegossen, das Produkt durch Absaugen isoliert und auf Isopopanol/Wasser umkristallisiert.

Man erhält 13,5 g (53 % der Theorie) 2-Chlor-3-(3-trifluormethyl-1-cyclohexyl-methyl)-1,4-naphtochinon vom Schmelzpunkt 104 °C.

Ausgangsstoffe der Formel (III)

Beispiel (III-1)

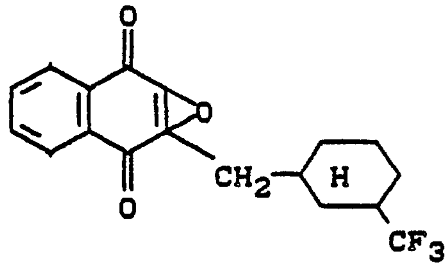

Eine Mischung aus 8 g (0,025 Mol) 2-(3-Trifluormethyl-1cyclohexyl-methyl)-1,4-naphthochinon, 2,7 g (0,025 Mol) Natriumcarbonat, 6,5 ml 30 %iges wäßrigem Hydrogenperoxid (entsprechend 0,057 Mol $H_2O_2$) und 100 ml Dioxan wird 10 Minuten bei 70 °C gerührt. Nach Abkühlen wird mit Wasser verdünnt und das kristallin angefallene Produkt durch Absaugen isoliert.

Man erhält 7,4 g (88 % der Theorie) 2,3-Epoxy-2,3-dihydro-2-(3-trifluormethyl-1-cyclohexyl-methyl)-1,4-naphthochinon vom Schmelzpunkt 85 °C.

Ausgangsstoffe der Formel (VII)

Beispiel (VII-1)

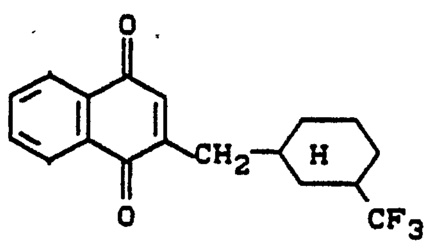

15,8 g (0,1 Mol) 1,4-Naphthochinon, 27,3 g (0,13 Mol) (3-Trifluormethyl-cyclohexyl)-essigsäure und 5,7 g (0,03 Mol) Silbernitrat werden in mit 150 ml Wasser und 150 ml Acetonitril vermischt und auf 65 °C erhitzt. Bei dieser Temperatur werden 34,2 g (0,15 Mol) Ammoniumperoxodisulfat als wäßrige Lösung innerhalb von 30 Minuten tropfenweise unter Rühren dazugegeben. Das Reaktionsgemisch wird weitere 30 Minuten bei 65 °C gerührt und dann auf Eis gegossen. Das Produkt wird durch Absaugen isoliert und durch Chromatographie an Kieselgel (mit Dichlormethan/Cyclohexan) gereinigt.

Man erhält 27,4 g (85 % der Theorie) 2-(3-Trifluormethyl-1-cyclohexyl-methyl)-1,4-naphthochinon vom Schmelzpunkt 65 °C.

**Ansprüche**

1. Schädlingsbekämpfungsmittel, gekennzeichnet durch einen Gehalt an mindestens einem substituierten 1,4-Naphthochinon der allgemeinen Formel (I)

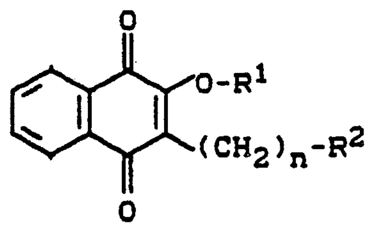

(I)

in welcher
n für die Zahlen Null oder 1 bis 12 steht,
$R^1$ für Wasserstoff oder für einen gegebenenfalls substituierten Rest R aus der Reihe Alkyl, Aralkyl, Alkylcarbonyl, (Hetero)Arylcarbonyl, Alkoxycarbonyl, Alkylsulfonyl und Arylsulfonyl steht und
$R^2$ für Halogenalkyl, gegebenenfalls substituiertes (Hetero)Aryl oder substituiertes Cycloalkyl steht.

2. Schädlingsbekämpfungsmittel gemäß Anspruch 1, gekennzeichnet durch einen Gehalt an mindestens einer Verbindung der Formel (I), in welcher
n für die Zahlen Null, 1 oder 2 steht,
$R^1$ für Wasserstoff oder für einen gegebenenfalls wie nachstehend angegeben substituierten Rest R aus der Reihe $C_1$-$C_{15}$-Alkyl (welches gegebenenfalls durch Fluor, Chlor oder $C_3$-$C_6$-Cycloalkyl substituiert ist), Benzyl (welches gegebenenfalls durch Fluor, Chlor oder Brom substituiert ist), $C_1$-$C_{15}$-Alkyl-carbonyl (welches gegebenenfalls durch Fluor, Chlor, Brom, Phenyl, $C_1$-$C_2$-Alkoxy oder $C_3$-$C_6$-Cycloalkyl substituiert ist), Benzoyl (welches gegebenenfalls durch Fluor, Chlor, Brom, Iod, Cyano, Nitro, $C_1$-$C_4$-Alkyl, $C_1$-$C_4$-Alkoxy, $C_1$-$C_4$-Halogenalkyl und/oder $C_1$-$C_4$-Halogenalkoxy substituiert ist), Furylcarbonyl oder Thienylcarbonyl (welche gegebenenfalls durch $C_1$-$C_4$-Alkyl substituiert sind), $C_1$-$C_4$-Alkoxy-carbonyl, $C_1$-$C_8$-Alkylsulfonyl (welches gegebenenfalls durch Fluor oder Chlor substituiert ist) und Phenylsulfonyl oder Naphthylsulfonyl (welche gegebenenfalls durch Fluor, Chlor, Brom, Cyano, Nitro, $C_1$-$C_4$-Alkyl, Trifluormethyl, $C_1$-$C_4$-Alkoxy, $C_1$-$C_4$-Alkylsulfonyl, Dimethylaminosulfonyl und/oder $C_1$-$C_4$-Alkoxycarbonyl substituiert sind) steht und
$R^2$ für durch Fluor und/oder Chlor substituiertes $C_1$-$C_8$-Alkyl, gegebenenfalls durch Fluor, Chlor, Brom, Cyano, Nitro, $C_1$-$C_6$-Alkyl, Trifluormethyl, $C_1$-$C_4$-Alkoxy, $C_1$-$C_2$-Fluoralkoxy, $C_1$-$C_4$-Alkylthio, $C_1$-$C_2$-Fluoralkylthio, $C_1$-$C_4$-Alkylsulfinyl, $C_1$-$C_2$-Fluoralkylsulfinyl, $C_1$- $C_4$-Alkylsulfonyl, $C_1$-$C_2$-Fluoralkylsulfonyl, Phenyl und/oder Tri-($C_1$-$C_2$-alkyl)-silyl substituiertes Phenyl, für Naphthyl, für gegebenenfalls durch $C_1$-$C_4$-Alkyl substituiertes Furyl oder Thienyl oder für durch $C_1$-$C_6$-Alkyl, Trifluormethyl, Cyclohexyl und/oder Tri($C_1$-$C_2$-alkyl)-silyl substituiertes Cyclohexyl steht.

3. Schädlingsbekämpfungsmittel gemäß Anspruch 1, gekennzeichente durch einen Gehalt an mindestens einer Verbindung der Formel (I), in welcher
n für die Zahlen Null oder 1 bis 4 steht,
$R^1$ für Wasserstoff, Methyl oder Acetyl steht und
$R^2$ für Perfluor-$C_1$-$C_6$-Alkyl, für durch Fluor, Chlor, $C_1$-$C_4$-Alkyl-Trifluormethyl, $C_1$-$C_3$-Alkoxy, Trifluormethoxy, $C_1$-$C_3$-Alkylthio, Trifluormethylsulfenyl, $C_1$-$C_3$-Alkylsulfinyl, Trifluormethylsulfinyl, $C_1$-$C_3$-Alkylsulfonyl, Trifluormethylsulfonyl oder Trimethylsilyl substituiertes Phenyl oder für durch $C_1$-$C_4$-Alkyl, Trifluormethyl oder Trimethylsilyl substituiertes Cyclohexyl steht.

4. Verfahren zur Bekämpfung von landwirtschaftlichen Schädlingen, insbesondere von Spinnentieren und Pilzen, dadurch gekennzeichnet, daß man substitu ierte 1,4-Naphthochinone der allgemeinen Formel (I) auf landwirtschaftliche Schädlinge und/oder deren Lebensraum einwirken läßt.

5. Verwendung von substituierten 1,4-Naphthochinonen der allgemeinen Formel (I) zur Bekämpfung von landwirtschaftlichen Schädlingen.

6. Verfahren zur Herstellung von Mitteln gegen landwirtschaftliche Schädlinge, dadurch gekennzeichnet, daß man substituierte 1,4-Naphthochinone gemäß Formel (I) mit Streckmitteln und/oder oberflächenaktiven Mitteln vermischt.

7. Substituierte 1,4-Naphthochinone der allgemeinen Formel (Ia)

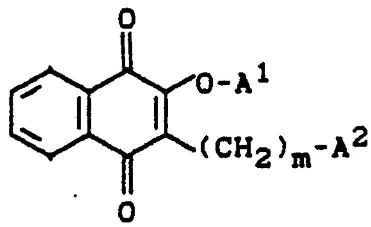

(Ia)

in welcher
m für die Zahlen 0 oder 1 bis 12 steht,
$A^1$ für Wasserstoff, Methyl, Benzyl, Acetyl, Methoxycarbonyl, Benzolsulfonyl oder Toluolsulfonyl steht und
$A^2$ für Perfluor-$C_1$-$C_6$-Alkyl, 2-Trifluormethyl-oder 3-Trifluormethyl-cyclohexyl, 2-Trifluormethyl- oder 3-Trifluormethyl-phenyl, 4-Trimethylsilyl-cyclohexyl oder 4-Trifluormethylthio-phenyl steht
- mit der Maßgabe, daß m von 0 und 2 verschieden ist, wenn $A^2$ für 3-Trifluormethyl-cyclohexyl oder 3-Trifluormethyl-phenyl steht.

8. Substituierte 1,4-Naphthochinone der Formel (Ia) gemäß Anspruch 7, dadurch gekennzeichnet, daß
m für die Zahlen Null oder 1 bis 10 steht,
$A^1$ für Wasserstoff, Methyl, Acetyl oder Benzyl steht und
$A^2$ für Perfluor-$C_1$-$C_6$-Alkyl, 2-Trifluormethyl-oder 3-Trifluormethyl-cyclohexyl, 2-Trifluormethyl- oder 3-Trifluormethyl-phenyl, 4-Trifluormethylsilyl-cyclohexyl oder 4-Trifluormethylthio-phenyl steht
- mit der Maßgabe, daß m von Null 2 und verschieden ist, wenn $A^2$ für 3-Trifluormethyl-cyclohexyl oder 3-Trifluormethyl-phenyl steht.

9. Verfahren zur Herstellung von substituierten 1,4-Naphthochinonen der allgemeinen Formel (Ia)

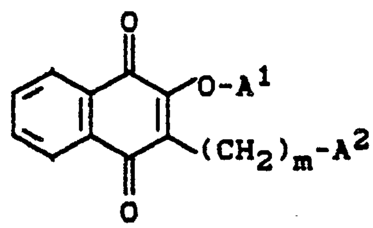

(Ia)

in welcher
m für die Zahlen 0 oder 1 bis 12 steht,
$A^1$ für Wasserstoff, Methyl, Benzyl, Acetyl, Methoxycarbonyl, Benzolsulfonyl oder Toluolsulfonyl steht und
$A^2$ für Perfluor-$C_1$-$C_6$-Alkyl, 2-Trifluormethyl-oder 3-Trifluormethyl-cyclohexyl 2-Trifluormethyl- oder 3-Trifluormethyl-phenyl, 4-Trimethylsilyl-cyclohexyl oder 4-Trifluormethylthio-phenyl steht
- mit der Maßgabe, daß m von 0 und 2 verschieden ist, wen $A^2$ für 3-Trifluormethyl-cyclohexyl oder 3-Trifluormethyl-phenyl steht,
dadurch gekennzeichnet, daß man

(a) zum Erhalt von Verbindungen der Formel (Ia), in welcher $A^1$ für Wasserstoff steht,
2-substituierte 3-Halogen-1,4-naphthochinone der allgemeinen Formel (II)

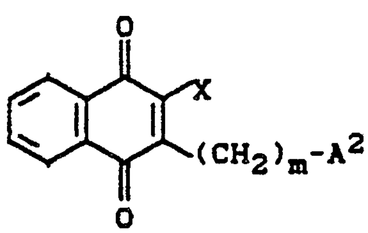

(II)

in welcher
m und $A^2$ die oben angegebenen Bedeutungen haben und
X für Halogen steht,

mit einem Alkalimetall- bzw. Erdalkalimetallhydroxid, gegebenenfalls in Gegenwart eines Verdünnungsmittels umsetzt, oder

(b) zum Erhalt von Verbindungen der Formel (Ia), in welcher $A^1$ für Wasserstoff steht, 2,3-Epoxy-2,3-dihydro-1,4-naphthochinone der allgemeinen Formel (III)

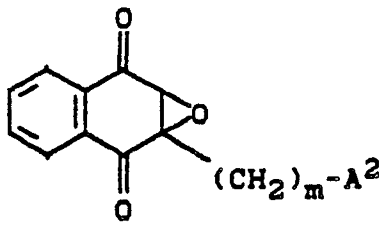

(III)

in welcher
m und $A^2$ die oben angegebenen Bedeutungen haben,
mit einem Alkalimetall- bzw. Erdalkalimetallhydroxid, gegebenenfalls in Gegenwart eines Verdünnungsmittels umsetzt, oder

(c) zum Erhalt von Verbindungen der Formel (Ia), in welcher $A^1$ für Methyl, Benzyl, Acetyl, Methoxycarbonyl, Benzolsulfonyl oder Toluolsulfonyl steht,
2-substituierte 3-Hydroxy-1,4- naphthochinone der allgemeinen Formel (Ib)

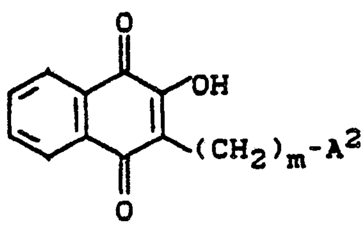

(Ib)

in welcher
m und $A^2$ die oben angegebenen Bedeutungen haben,
mit Verbindungen der allgemeinen Formel (IV)

Y - A (IV)

in welcher
A für Methyl, Benzyl, Acetyl, Methoxycarbonyl, Benzolsulfonyl oder Toluolsulfonyl steht und
Y für eine nucleofuge Abgangsgruppe, vorzugsweise Halogen, steht,
gegebenenfalls in Gegenwart eines Metallsalzes, gegebenenfalls in Gegenwart eines Säureakzeptors und gegebenenfalls in Gegenwart eines Verdünnungsmittels umsetzt.

Europäisches Patentamt

# EUROPÄISCHER RECHERCHENBERICHT

Nummer der Anmeldung

EP 88 10 9993

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | FR-A-2 085 660 (PFIZER INC)<br>* Seite 7, Zeilen 8-26 * | 7 | C 07 C 50/32<br>C 07 C 46/00<br>C 07 C 69/96<br>C 07 C 69/145<br>C 07 C 143/68<br>C 07 F 7/08<br>A 01 N 35/06<br>A 01 N 37/02<br>A 01 N 41/04 |
| A | EP-A-0 123 238 (THE WELLCOME FOUNDATION LIMITED)<br>* Patentanspruch 1; Seite 5, Zeile 3; Seiten 15,16; Beispiel 8 * | 7 | |
| A | US-A-2 553 647 (L.F. FIESER et al.)<br>* Spalten 1-10 * | 7 | |
| A | US-A-4 055 661 (R.F. BELLINA et al.)<br>* Patentansprüche * | 1 | |
| A | US-A-4 110 473 (R. BENSON et al.)<br>* Patentansprüche * | 1 | |
| A | US-A-3 673 222 (S. ARCHER et al.)<br>* Spalte 6, Zeile 66 - Spalte 7, Zeile 2 * | 8 | |
| | | | **RECHERCHIERTE SACHGEBIETE (Int. Cl.4)** |
| | | | C 07 C 50/00<br>C 07 C 46/00<br>C 07 C 69/00<br>C 07 C 143/00 |

Der vorliegende Recherchenbericht wurde für alle Patentansprüche erstellt

| Recherchenort | Abschlußdatum der Recherche | Prüfer |
|---|---|---|
| DEN HAAG | 31-10-1988 | BONNEVALLE E.I.H. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)